# EUROPEAN PATENT APPLICATION

(11) **EP 4 748 931 A1**
(43) Date of publication of application: **27.05.2026**
(21) Application number: 24863162.4
(22) Date of filing: 03.09.2024
(51) Int. Cl.: C12N 9/88, C12N 15/70, A23K 10/14, A23K 10/16

(54) **NOVEL POLYPEPTIDE EXHIBITING DON-DEGRADING ACTIVITY**

(30) Priority: 04.09.2023 KR 20230116946
(71) Applicant: CJ Cheiljedang Corporation, Seoul 04560 (KR)
(72) Inventor: KIM, Nam Yoon, Seoul 04560 (KR); LEE, Seung-hee, Seoul 04560 (KR); YANG, Tae Joo, Seoul 04560 (KR); KANG, Young Seo, Seoul 04560 (KR)
(74) Representative: Meissner Bolte Partnerschaft mbB
(86) International application number: PCT/KR2024/013258
(87) International publication number: WO 2025/053581

(57) **Abstract**

Provided is a modified polypeptide having DON degradation activity.

## Description

### [Technical Field]

The present disclosure relates to a modified polypeptide having deoxynivalenol (DON) degradation activity.

### [Background Art]

Mycotoxins are a general term for metabolites derived from fungi, which are toxic to animals. When crops or stored agricultural products are infected or contaminated with fungi that produce mycotoxins, these mycotoxins are produced and pose a health risk to animals or humans. Trichothecenes, often found in grains, are sesquiterpenoid mycotoxins with a structure of 12,13-epoxi-trichothec-9-ene, and are produced primarily by *Fusarium*, *Myrothecium*, *Stachybotrys*, and *Trichothecium. Fusarium* is the most important trichothecene-producing species from an economic point of view, and among the toxins, the most commonly detected toxins in grains are deoxynivalenol (DON), nivalenol (NIV), and acetylated derivatives thereof.

Trichothecenes act on eukaryotic cells to exhibit various detrimental effects, such as inhibition of protein synthesis, cytotoxicity, cell death, etc. When feeds or foods contaminated with DON are consumed, humans experience symptoms such as immunosuppression, anemia, headache, nausea, abdominal pain, etc., while animals experience symptoms such as refusal to eat, vomiting, growth retardation, reproductive disorders, etc. Due to the effects of global warming, it is expected that the annual exposure level of humans and animals to trichothecene will exceed the tolerable level.

Most countries have laws regulating the allowable levels of DON in feeds, foods, and harvested grains. Although efforts have been made to reduce DON contamination through pesticides targeting *Fusarium* spp. or breeding, DON still contaminates grains. In order to detoxify DON, various chemicals, such as ozone, ammonia, chlorine, hydrogen peroxide, sodium bisulfite, etc., have been treated, but they are not being scaled up due to cost, safety issues, negative effects on grain quality, etc. The most effective method is to treat with sodium metabisulfite. However, in Europe, this is prohibited for use in food grains, and accordingly, there is a need for a safe method of detoxifying DON.

### [Prior Art Document]

### [Non-Patent Document]

S Chakraborty et al, Virulence. 2015 Jan; 6(1): 50-65 (2014.12.17), Lactoylglutathione lyase, a critical enzyme in methylglyoxal detoxification, contributes to survival of Salmonella in the nutrient rich environment.

### [Disclosure]

### [Technical Problem]

The present inventors developed a modified polypeptide having deoxynivalenol (DON) degradation activity.

### [Technical Solution]

An object of the present disclosure is to provide a modified polypeptide having deoxynivalenol (DON) degradation activity, wherein amino acids at positions corresponding to any one or more positions selected from positions 28, 29, 87, 91, 101, and 152 of SEQ ID NO: 1 are substituted with different amino acids.

Another object of the present disclosure is to provide a polynucleotide encoding the modified polypeptide of the present disclosure.

Still another object of the present disclosure is to provide a microorganism comprising any one or more of the modified polypeptide; and a polynucleotide encoding the modified polypeptide.

Still another object of the present disclosure is to provide a composition for degrading deoxynivalenol (DON), the composition comprising any one or more of the modified polypeptide; and a microorganism expressing the modified polypeptide.

Still another object of the present disclosure is to provide a feed additive composition comprising any one or more of the modified polypeptide; and a microorganism expressing the modified polypeptide.

Still another object of the present disclosure is to provide a method of degrading deoxynivalenol (DON), the method comprising the step of reacting DON with any one or more of the modified polypeptide; and a microorganism expressing the modified polypeptide.

Still another object of the present disclosure is to provide a method of preparing a feed product, the method comprising the step of mixing a feed component with a feed additive composition comprising any one or more of the modified polypeptide; and a microorganism expressing the modified polypeptide.

Still another object of the present disclosure is to provide a method of preparing the modified polypeptide having DON degradation activity, the method comprising the steps of:
culturing a microorganism comprising any one or more of the modified polypeptide; and a polynucleotide encoding the modified polypeptide; and
recovering the modified polypeptide having DON degradation activity of the present disclosure, which is expressed in the culturing step.

### [Advantageous Effects]

A modified polypeptide having DON degradation activity of the present disclosure, in which DON degradation activity, thermal stability, thermal tolerance, and acid resistance are improved, may be usefully applied to a variety of industrial fields.

### [Brief Description of Drawings]

FIG. 1 shows an examination of DON degradation activity of a parent DON degradation enzyme (GhSPG-216M2; SEQ ID NO: 1) and a modified polypeptide having DON degradation activity (GhSPG-216M3-8) of the present disclosure;
FIG. 2 shows an examination of thermal stability of the parent DON degradation enzyme and the modified polypeptide having DON degradation activity of the present disclosure;
FIG. 3 shows an examination of acid resistance of the parent DON degradation enzyme and the modified polypeptide having DON degradation activity of the present disclosure;
FIG. 4 shows a comparative evaluation of activity of variants, each in which an amino acid at position 101 of the parent DON degradation enzyme was substituted with a different amino acid;
FIG. 5 shows a comparative evaluation of thermal tolerance of variants, each in which an amino acid at position 101 of the parent DON degradation enzyme was substituted with a different amino acid;
FIG. 6 shows a comparative evaluation of acid resistance of variants, each in which an amino acid at position 101 of the parent DON degradation enzyme was substituted with a different amino acid; and
FIG. 7 shows an examination of DON degradation activity of the parent DON degradation enzyme and the modified polypeptide having DON degradation activity of the present disclosure.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those of ordinary skill in the art may be able to recognize or confirm, using only conventional experimentation, many equivalents to the particular aspects of the disclosure described herein. Furthermore, it is also intended that these equivalents be comprised in the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a modified polypeptide having deoxynivalenol (DON) degradation activity, wherein amino acids at positions corresponding to any one or more positions selected from positions 28, 29, 87, 91, 101, and 152 of SEQ ID NO: 1 are substituted with different amino acids.

As used herein, the "deoxynivalenol (DON) degradation activity" means catalyzing the degradation of deoxynivalenol (DON) by converting the same into an iso-DON form.

In the present disclosure, deoxynivalenol (DON) has a chemical formula of C₁₅H₂₀O₆ and a molecular weight of 296.3 g/mol (CAS NO.:51481-10-8), and may be a mycotoxin belonging to Type B trichothecene, which is one of the epoxy-sesquiterpenoids, and is also called vomitoxin. Deoxynivalenol may be produced by fungi that cause diseases in plants, of which the genus *Fusarium* is a known producer, and when consumed, symptoms such as immunosuppression, anemia, headache, nausea, abdominal pain, etc. occur, and in animals, symptoms such as refusal to eat, vomiting, growth inhibition, and reproductive disorders, etc. may occur.

Meanwhile, the degradation of deoxynivalenol (DON) may be used interchangeably with detoxification of DON, inactivation of DON, and removal of DON contamination (decontaminating).

Although the modified polypeptide having DON degradation activity provided in the present disclosure is defined as a polypeptide having substitutions of amino acids at positions corresponding to any one or more positions selected from positions 28, 29, 87, 91, 101, and 152 of SEQ ID NO: 1, the addition of a meaningless sequence upstream or downstream of the amino acid sequence of SEQ ID NO: 1, a naturally occurring mutation, or a silent mutation thereof is not excluded, and it will be apparent to those skilled in the art that as long as a protein has activity identical or corresponding to the activity of the protein consisting of the amino acid sequence of SEQ ID NO: 1, it may belong to the polypeptide having DON degradation activity provided in the present disclosure.

In other words, although described as 'a protein or polypeptide having an amino acid sequence represented by a specific SEQ ID NO' or 'a protein or polypeptide comprising an amino acid sequence represented by a specific SEQ ID NO' in the present disclosure, it is obvious that any protein having an amino acid sequence with deletion, modification, substitution, or addition in part of the sequence may also be used in the present disclosure, as long as the protein may have an activity identical or corresponding to that of the polypeptide consisting of the amino acid sequence of the corresponding SEQ ID NO.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and comprise sequences encoding any wild-type polypeptides.

As used herein, a parent sequence or backbone refers to a reference sequence in which modification is introduced to be a modified polypeptide. In other words, the parent sequence may serve as a starting sequence in which mutations such as substitutions, insertions, and/or deletions may be introduced. The parent sequence may be naturally occurring or wild-type, or a variant in which one or more substitutions, insertions, or deletions have occurred in the natural or wild-type, or may be an artificially synthesized sequence. When the parent sequence is an amino acid sequence exhibiting activity, i.e., an amino acid sequence of an enzyme, it may be referred to as a parent enzyme.

With respect to amino acid or nucleic acid sequences in the present disclosure, the term "fragment" means a part of a parent sequence. For example, it may be a polypeptide in the form where one or more amino acids are removed from the C- or N-terminus of the parent sequence.

As used herein, the term "fragment" of an enzyme may refer to a "functional fragment". The "functional fragment" may also be referred to as an active fragment, and refers to a polypeptide that is a part of a parent enzyme and has enzymatic activity of the parent enzyme. For example, the functional fragment of an enzyme may comprise a catalytic site of the enzyme.

The fragment of the enzyme may comprise a part of the full length of the parent enzyme. For example, the fragment of the enzyme may comprise amino acids of at least about 20%, 30%, 40%, 50%, 60%, 70%, 80%, 90%, 95%, 98%, 99% or more, or less than 100% of the full length of the parent enzyme, but is not limited thereto.

As used herein, the term "modifying" means changing or altering. This may be altering from a naturally occurring state. For example, an enzyme may be altered in such a way that the enzyme is altered from a parent or reference sequence.

In the present disclosure, a modified enzyme may be an enzyme that does not exist as it is in nature, i.e., a non-naturally occurring enzyme.

As used herein, the term "modified" means being altered, e.g., from its naturally occurring form. The modified enzyme of the present disclosure comprises non-naturally occurring enzymes or naturally occurring variants. For example, the modified enzyme of the present disclosure is a modified enzyme that has not been found in nature. For example, the modified enzyme of the present disclosure may be a non-spontaneously occurring enzyme, but is not limited thereto.

As used herein, the term "modification", when used with respect to amino acid/nucleic acid sequences, may comprise substitution of an amino acid/nucleic acid residue of a parent sequence with a different amino acid/nucleic acid residue in one or more positions in an amino acid sequence, deletion of an amino acid/nucleic acid residue (or series of amino acid/nucleic acid residues) from a parent sequence in one or more positions, insertion of an amino acid/nucleic acid residue (or series of amino acid/nucleic acid residues) into a parent sequence in one or more positions, truncation of the N-terminal and/or C-terminal amino acid sequences or 5' and/or 3' nucleic acid sequences, and any combination thereof.

As used herein, the term "variant" or "modified polypeptide" of an enzyme refers to a protein having one or more amino acids different from the parent enzyme by conservative substitution and/or modification. The "variant" or "modified polypeptide" may be used interchangeably. The variant or modified polypeptide may be non-naturally occurring, but is not limited thereto.

The variant differs from the sequence of the parent enzyme by one or more modifications, e.g., substitutions, deletions, and/or insertions of amino acids.

Such variants may be generally identified by modifying one or more amino acids in the parent enzyme and evaluating the properties of the modified protein. That is, the ability of the variants may be enhanced, unchanged, or reduced, as compared to the parent enzyme.

Further, some variants may comprise modified polypeptides in which one or more regions, such as an N-terminal leader sequence or transmembrane domain, have been removed.

Other variants may comprise variants in which a part has been removed from the N- and/or C-terminal end of a mature protein.

The term "variant" or "modified polypeptide" may be used interchangeably with terms such as modification, modified protein, mutant, mutein, divergent, variant, etc., and is not limited as long as the terms are used to indicate mutation.

The variants may also comprise deletion or addition of amino acids that have minimal influence on the properties and secondary structure of a polypeptide. For example, the polypeptides may be conjugated with a signal (or leader) sequence at the N-terminal end which is involved in the translocation of proteins co-translationally or post-translationally. Further, the polypeptides may also be conjugated with another sequence or linker to identify, purify, or synthesize the polypeptides.

As used herein, the term "conservative substitution" refers to the substitution of an amino acid with another amino acid having similar structural and/or chemical properties. Such amino acid substitution may generally occur based on the similarity of polarity, charge, solubility, hydrophobicity, hydrophilicity, and/or amphipathic nature of a residue.

As used herein, the "parent DON degradation enzyme" refers to a DON degradation enzyme to which a modification is applied in order to produce the variant or the modified polypeptide of the present disclosure. Specifically, the parent DON degradation enzyme, parent enzyme, or parent sequence may be a naturally occurring polypeptide or a wild-type polypeptide, or may be a mature polypeptide thereof, and may comprise a variant or functional fragment thereof, but is not limited thereto, as long as the polypeptide has DON degradation activity and may be a parent of the variant.

The parent DON degradation enzyme provided in the present disclosure may be a polypeptide of SEQ ID NO: 1, but is not limited thereto. Further, it may be a polypeptide having about 80%, 85%, 90%, 95%, 96%, 97%, 98%, or 99% or more sequence identity to the polypeptide of SEQ ID NO: 1 as long as it has DON degradation activity, and any polypeptide may be comprised within the scope of the parent DON degradation enzyme as long as it has the same or corresponding activity as the polypeptide consisting of the amino acid sequence of SEQ ID NO: 1.

The parent DON degradation enzyme of the variant provided in the present disclosure may be derived from the genus *Gossypium,* the genus *Handroanthus,* or the genus *Hibiscus.* Specifically, it may be derived from the genus *Gossypium.*

In the present disclosure, the "modified polypeptide having DON degradation activity" may be a variant of the parent DON degradation enzyme.

As used herein, the term "variant of the parent DON degradation enzyme" or "modified polypeptide having DON degradation activity" refers to a protein having DON degradation activity, in which one or more amino acids are different from the amino acid sequence of the parent DON degradation enzyme.

The "modified polypeptide having DON degradation activity", the "variant of the parent DON degradation enzyme", and the "DON degradation enzyme variant" may be used interchangeably with each other.

The modified polypeptide provided in the present disclosure may comprise modification of one or more amino acids in the sequence of the parent DON degradation enzyme, while having DON degradation activity. The modification may be substitution of amino acids and/or formation of disulfide bonds.

Specifically, the variant provided in the present disclosure may have one or more altered functions or properties compared to the parent DON degradation enzyme by comprising modification of one or more amino acids in the sequence of the parent DON degradation enzyme, while having DON degradation activity.

In one specific embodiment, the variant provided in the present disclosure may have one or more altered functions or properties compared to the parent DON degradation enzyme by comprising modification of one or more amino acids in the sequence of the parent DON degradation enzyme, and may have one or more conservative substitutions, while having DON degradation activity.

The variant provided in the present disclosure, which is a variant of the parent DON degradation enzyme, may be a polypeptide having DON degradation activity. In one specific embodiment, the variant provided in the present disclosure may comprise modification at positions corresponding to any one or more positions selected from positions 28, 29, 87, 91, 101, and 152 of SEQ ID NO: 1.

In the present disclosure, the position number refers to a position corresponding to the position of the polypeptide of SEQ ID NO: 1, and the term "corresponding" is as described above.

In one specific embodiment, the modified polypeptide of the present disclosure may comprise substitutions of amino acids at positions corresponding to any one or more positions selected from positions 28, 29, 87, 91, 101, and 152 of SEQ ID NO: 1, as compared to the amino acid sequence of SEQ ID NO: 1, but is not limited thereto.

For a specific example thereof, the modified polypeptide provided in the present disclosure may comprise substitutions of combinations of 2, 3, 4, 5, or 6 positions selected from the positions with different amino acids.

In any one specific embodiment of the above-described specific embodiments, the modified polypeptide may comprise modification of amino acids at positions selected from the following i) to Ixiii):
i) position 28; ii) position 29; iii) position 87; iv) position 91; v) position 101; vi) position 152; vii) positions 28+29; viii) positions 28+87; ix) positions 28+91; x) positions 28+101; xi) positions 28+152; xii) positions 29+87; xiii) positions 29+91; xiv) positions 29+101; xv) positions 29+152; xvi) positions 29+152; xvii) positions 87+ 91; xviii) positions 87+ 101; xix) positions 87+ 152; xx) positions 91+ 101; xxi)91+ 152; xxii) positions 101+ 152; xxiii) positions 28+ 29+ 87; xxiv) positions 28+ 29+ 101; xxv)28+ 29+ 152; xxvi) positions 28+ 87+ 91; xxvii) positions 28+ 87+ 101; xxviii) positions 28+ 87+ 152; xxix) positions 28+ 91+ 101; xxx)28+ 91+ 152; xxxi)28+ 101+ 152 ; xxxii)29+ 87+ 91; xxxiii)29+ 87+ 101; xxxiv)29+ 87+ 152; xxxv)28+ 91+ 101; xxxvi)29+ 91+ 152; xxxvii) positions 29+ 101+ 152; xxxviii) positions 87+ 91+ 101; xxxix) positions 87+ 91+ 152; xl) positions 87+ 101+ 152; xli)91+ 101+ 152; xlii) positions 28+ 29+ 87+ 91; xliii) positions 28+ 29+ 87+ 101; xliv) positions 28+ 29+ 87+ 152; xlv) positions 28+ 29+ 91+ 101; xlvi) positions 28+ 29+ 91+ 152; xlvii) positions 28+ 29+ 101+ 152; xlviii) positions 28+ 87+ 91+ 101; xlix) positions 28+ 87+ 91+ 152; l) positions 28+ 87+ 101+ 152; li)28+ 91+ 101+ 152; lii)29+ 87+ 91+ 101; liii) positions 29+ 87+ 91+ 152; liv)29+ 87+ 101+ 152; lv)29+ 91+ 101+ 152; lvi)87+ 91+ 101+ 152; lvii) positions 28+ 29+ 87+ 91+ 101; lviii) positions 28+ 29+ 87+ 91+ 152; lix) positions 28+ 29+ 87+ 101+ 152; lx) positions 28+ 29+ 91+ 101+ 152; lxi) positions 28+ 87+ 91+ 101+ 152; lxii) positions 29+ 87+ 91+ 101+ 152; and lxiii) positions 28+ 29+ 87+ 91+ 101+ 152;

Herein, the position number refers to a position corresponding to the position of the polypeptide of SEQ ID NO: 1.

In any one specific embodiment of the above-described specific embodiments, the modified polypeptide provided in the present disclosure may comprise substitutions of amino acids at positions corresponding to any one or more positions selected from positions 28, 29, 87, 91, 101, and 152 of SEQ ID NO: 1 with G, A, V, L, I, M, F, W, P, S, T, C, Y, N, Q, D, E, K, R, or H.

In any one specific embodiment of the above-described specific embodiments, the modified polypeptide provided in the present disclosure may comprise substitutions of amino acids at positions corresponding to any one or more positions selected from positions 28, 29, 87, 91, 101, and 152 of SEQ ID NO: 1 with polar, nonpolar, hydrophobic, or acidic amino acids.

Further, the modified polypeptide provided in the present disclosure may comprise a substitution of an amino acid corresponding to position 28 of SEQ ID NO: 1 with isoleucine.

Further, the modified polypeptide provided in the present disclosure may comprise a substitution of an amino acid corresponding to position 29 of SEQ ID NO: 1 with leucine.

Further, the modified polypeptide provided in the present disclosure may comprise a substitution of an amino acid corresponding to position 87 of SEQ ID NO: 1 with isoleucine.

Further, the modified polypeptide provided in the present disclosure may comprise a substitution of an amino acid corresponding to position 91 of SEQ ID NO: 1 with serine.

Further, the modified polypeptide provided in the present disclosure may comprise a substitution of an amino acid corresponding to position 101 of SEQ ID NO: 1 with phenylalanine.

Further, the modified polypeptide provided in the present disclosure may comprise a substitution of an amino acid corresponding to position 152 of SEQ ID NO: 1 with glycine.

Further, the modified polypeptide provided in the present disclosure may comprise a substitution of the amino acid corresponding to position 28 of SEQ ID NO: 1 with isoleucine; and a substitution of the amino acid corresponding to position 101 of SEQ ID NO: 1 with phenylalanine.

Further, the modified polypeptide provided in the present disclosure may comprise a substitution of the amino acid corresponding to position 28 of SEQ ID NO: 1 with isoleucine; and a substitution of the amino acid corresponding to position 152 of SEQ ID NO: 1 with glycine.

Further, the modified polypeptide provided in the present disclosure may comprise a substitution of the amino acid corresponding to position 28 of SEQ ID NO: 1 with isoleucine; a substitution of the amino acid corresponding to position 101 of SEQ ID NO: 1 with phenylalanine; and a substitution of the amino acid corresponding to position 152 of SEQ ID NO: 1 with glycine.

In any one specific embodiment of the above-described specific embodiments, the modified polypeptide may comprise any one or more substitutions selected from the following substitutions:

| |
|---|
| F28I |
| K101F, K101G, K101A, K101V, K101L, K101I, K101M, K101W, K101P, K101S, K101T, K101C, K101Y, K101N, K101Q, K101D, K101E, K101R or K101H |
| D152G |
| M29L |
| A87I |
| G91S |
| F28I+M29L |
| F28I+A87I |
| F28I+G91S |
| F28I+K101F(K101G, K101A, K101V, K101L, K101I, K101M, K101W, K101P, K101S, K101T, K101C, K101Y, K101N, K101Q, K101D, K101E, K101R or K101H) |
| F28I+D152G |
| M29L+A87I |
| M29L+G91S |
| M29L+K101F(K101G, K101A, K101V, K101L, K101I, K101M, K101W, K101P, K101S, K101T, K101C, K101Y, K101N, K101Q, K101D, K101E, K101R or K101H) |
| M29L+D152G |
| M29L+D152G |
| A87I+ G91S |
| A87I+ K101F(K101G, K101A, K101V, K101L, K101I, K101M, K101W, K101P, K101S, K101T, K101C, K101Y, K101N, K101Q, K101D, K101E, K101R or K101H) |
| A87I+ D152G |
| G91S+ K101F(K101G, K101A, K101V, K101L, K101I, K101M, K101W, K101P, K101S, K101T, K101C, K101Y, K101N, K101Q, K101D, K101E, K101R or K101H) |
| G91S+ D152G |
| K101F(K101G, K101A, K101V, K101L, K101I, K101M, K101W, K101P, K101S, K101T, K101C, K101Y, K101N, K101Q, K101D, K101E, K101R or K101H)+ D152G |
| F28I+ M29L+ A87I |
| F28I+ M29L+ K101F(K101G, K101A, K101V, K101L, K101I, K101M, K101W, K101P, K101S, K101T, K101C, K101Y, K101N, K101Q, K101D, K101E, K101R or K101H) |
| F28I+ M29L+ D152G |
| F28I+ A87I+ G91S |
| F28I+ A87I+ K101F(K101G, K101A, K101V, K101L, K101I, K101M, K101W, K101P, K101S, K101T, K101C, K101Y, K101N, K101Q, K101D, K101E, K101R or K101H) |
| F28I+ A87I+ D152G |
| F28I+ G91S+ K101F(K101G, K101A, K101V, K101L, K1011, K101M, K101W, K101P, K101S, K101T, K101C, K101Y, K101N, K101Q, K101D, K101E, K101R or K101H) |
| F28I+ G91S+ D152G |
| F28I+ K101F(K101G, K101A, K101V, K101L, K101I, K101M, K101W, K101P, K101S, K101T, K101C, K101Y, K101N, K101Q, K101D, K101E, K101R or K101H)+ D152G |
| M29L+ A87I+ G91S |
| M29L+ A87I+ K101F(K101G, K101A, K101V, K101L, K1011, K101M, K101W, K101P, K101S, K101T, K101C, K101Y, K101N, K101Q, K101D, K101E, K101R or K101H) |
| M29L+ A87I+ D152G |
| F281+G91S+ K101F(K101G, K101A, K101V, K101L, K101I, K101M, K101W, K101P, K101S, K101T, K101C, K101Y, K101N, K101Q, K101D, K101E, K101R or K101H) |
| M29L+ G91S+ D152G |
| M29L+ K101F(K101G, K101A, K101V, K101L, K101I, K101M, K101W, K101P, K101S, K101T, K101C, K101Y, K101N, K101Q, K101D, K101E, K101R or K101H)+ D152G |
| A87I+ G91S+ K101F(K101G, K101A, K101V, K101L, K1011, K101M, K101W, K101P, K101S, K101T, K101C, K101Y, K101N, K101Q, K101D, K101E, K101R or K101H) |
| A87I+ G91S+ D152G |
| A87I+ K101F(K101G, K101A, K101V, K101L, K101I, K101M, K101W, K101P, K101S, K101T, K101C, K101Y, K101N, K101Q, K101D, K101E, K101R or K101H)+ D152G |
| G91S+ K101F(K101G, K101A, K101V, K101L, K101I, K101M, K101W, K101P, K101S, K101T, K101C, K101Y, K101N, K101Q, K101D, K101E, K101R or K101H)+ D152G |
| F28I+ M29L+ A87I+ G91S |
| F28I+ M29L+ A87I+ K101F(K101G, K101A, K101V, K101L, K101I, K101M, K101W, K101P, K101S, K101T, K101C, K101Y, K101N, K101Q, K101D, K101E, K101R or K101H) |
| F28I+ M29L+ A87I+ D152G |
| F28I+ M29L+ G91S+ K101F(K101G, K101A, K101V, K101L, K101I, K101M, K101W, K101P, K101S, K101T, K101C, K101Y, K101N, K101Q, K101D, K101E, K101R or K101H) |
| F28I+ M29L+ G91S+ D152G |
| F28I+ M29L+ K101F(K101G, K101A, K101V, K101L, K101I, K101M, K101W, K101P, K101S, K101T, K101C, K101Y, K101N, K101Q, K101D, K101E, K101R or K101H)+ D152G |
| F28I+ A87I+ G91S+ K101F(K101G, K101A, K101V, K101L, K101I, K101M, K101W, K101P, K101S, K101T, K101C, K101Y, K101N, K101Q, K101D, K101E, K101R or K101H) |
| F28I+ A87I+ G91S+ D152G |
| F28I+ A87I+ K101F(K101G, K101A, K101V, K101L, K101I, K101M, K101W, K101P, K101S, K101T, K101C, K101Y, K101N, K101Q, K101D, K101E, K101R or K101H)+ D152G |
| F28I+ G91S+ K101F(K101G, K101A, K101V, K101L, K1011, K101M, K101W, K101P, K101S, K101T, K101C, K101Y, K101N, K101Q, K101D, K101E, K101R or K101H)+ D152G |
| M29L+ A87I+ G91S+ K101F(K101G, K101A, K101V, K101L, K1011, K101M, K101W, K101P, K101S, K101T, K101C, K101Y, K101N, K101Q, K101D, K101E, K101R or K101H) |
| M29L+ A87I+ G91S+ D152G |
| M29L+ A87I+ K101F(K101G, K101A, K101V, K101L, K1011, K101M, K101W, K101P, K101S, K101T, K101C, K101Y, K101N, K101Q, K101D, K101E, K101R or K101H)+ D152G |
| M29L+ G91S+ K101F(K101G, K101A, K101V, K101L, K101I, K101M, K101W, K101P, K101S, K101T, K101C, K101Y, K101N, K101Q, K101D, K101E, K101R or K101H)+ D152G |
| A87I+ G91S+ K101F(K101G, K101A, K101V, K101L, K1011, K101M, K101W, K101P, K101S, K101T, K101C, K101Y, K101N, K101Q, K101D, K101E, K101R or K101H)+ D152G |
| F28I+ M29L+ A87I+ G91S+ K101F(K101G, K101A, K101V, K101L, K101I, K101M, K101W, K101P, K101S, K101T, K101C, K101Y, K101N, K101Q, K101D, K101E, K101R or K101H) |
| F28I+ M29L+ A87I+ G91S+ D152G |
| F28I+ M29L+ A87I+ K101F(K101G, K101A, K101V, K101L, K101I, |
| K101M, K101W, K101P, K101S, K101T, K101C, K101Y, K101N, K101Q, K101D, K101E, K101R or K101H)+ D152G |
| F28I+ M29L+ G91S+ K101F(K101G, K101A, K101V, K101L, K101I, K101M, K101W, K101P, K101S, K101T, K101C, K101Y, K101N, K101Q, K101D, K101E, K101R or K101H)+ D152G |
| F28I+ A87I+ G91S+ K101F(K101G, K101A, K101V, K101L, K101I, K101M, K101W, K101P, K101S, K101T, K101C, K101Y, K101N, K101Q, K101D, K101E, K101R or K101H)+ D152G |
| M29L+ A87I+ G91S+ K101F(K101G, K101A, K101V, K101L, K1011, K101M, K101W, K101P, K101S, K101T, K101C, K101Y, K101N, K101Q, K101D, K101E, K101R or K101H)+ D152G |
| F281+ M29L+ A87I+ G91S+ K101F(K101G, K101A, K101V, K101L, K101I, K101M, K101W, K101P, K101S, K101T, K101C, K101Y, K101N, K101Q, K101D, K101E, K101R or K101H)+ D152G |

In any one specific embodiment of the above-described specific embodiments, the modified polypeptide provided in the present disclosure may comprise SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33, or SEQ ID NO: 35.

As used herein, the term "corresponding to" refers to an amino acid residue at the position recited in a protein or polypeptide, or an amino acid residue which is similar, identical, or homologous to the residue recited in a protein or polypeptide. Identifying an amino acid at a corresponding position may be determining a particular amino acid in a sequence that refers to a particular sequence. As used herein, the "corresponding region" generally refers to a similar or corresponding position in the related protein or reference protein.

In the present disclosure, SEQ ID NO: 1 may be used as a reference sequence to determine the position of amino acids in any amino acid sequence.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

That is, SEQ ID NO: 1 disclosed herein may be used to determine the corresponding amino acid residues in any polypeptide having DON degradation activity. Unless indicated otherwise in the present disclosure, residues of a particular amino acid sequence are numbered based on SEQ ID NO: 1.

For example, any amino acid sequence is aligned with SEQ ID NO: 1, and based on the alignment, each amino acid residue of the amino acid sequence may be numbered with reference to the numerical position of the amino acid residue corresponding to the amino acid residue of SEQ ID NO: 1. For example, a sequence alignment algorithm such as that described herein may identify the position of an amino acid or a position where modifications such as substitutions, insertions or deletions occur, compared to a query sequence (also referred to as a "reference sequence").

In such alignment, the Needleman-Wunsch algorithm (Needleman and Wunsch, 1970, J. Mol. Biol. 48: 443-453), the Needle program of the EMBOSS package (EMBOSS: The European Molecular Biology Open Software Suite, Rice et al., 2000), Trends Genet. 16: 276-277), etc. may be used, but are not limited thereto.

Further, the corresponding amino acid residue in another polypeptide having DON degradation activity may be identified by multiple sequence alignment. Examples of the multiple sequence alignment known in the art comprise programs such as MUSCLE (multiple sequence comparison by log-expectation; version 3.5 or later; Edgar, 2004, Nucleic Acids Research 32: 1792-1797), MAFFT (6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), and EMBOSS EMMA employing ClustalW (1.83 or later; Thompson et al., 1994, Nucleic Acids Research 22 : 4673-4680), and their respective default parameters may be used, but are not limited thereto.

In addition, when enzymes diverged from the mature polypeptide of SEQ ID NO: 1 fail to detect their relationship by traditional sequence-based comparison, other pairwise sequence comparison algorithms may be used (Lindahl and Elofsson, 2000, J. Mol. Biol. 295: 613-615). Higher sensitivity in sequence-based searching may be attained using search programs that utilize probabilistic representations of polypeptide families (profiles) to search databases. For example, the PSI BLAST program generates profiles through an iterative database search process and is capable of detecting remote homologs (Atschul et al., 1997, Nucleic Acids Res. 25: 3389-3402). Even greater sensitivity may be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources, such as PSI BLAST, secondary structure prediction, structural alignment profiles, and solvation potentials, as input to a neural network that predicts the structural fold for a query sequence. Similarly, the method of Gough et al., 2000, J. Mol. Biol. 313: 903-919 may be used to align a sequence of unknown structure with the superfamily models present in the SCOP database. These alignments may be used in sequence to generate homology models for the polypeptides, and such models may be assessed for accuracy using a variety of tools developed for that purpose.

For proteins of known structure, several tools and resources are available for retrieving and generating structural alignments. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or CE (Combinatorial extension) (Shindyalov and Bourne, 1998, Protein Engineering 11: 739-747). Implementation of these algorithms may be additionally utilized to query structure databases with a structure of interest in order to discover possible structural homologs (Holm and Park, 2000, Bioinformatics 16: 566-567).

The above methods are illustrative, and are not limited thereto.

Throughout the entire specification of the present disclosure, the conventional one-letter and three-letter codes for naturally occurring amino acids are used. Further, the amino acids abbreviated herein are described according to the nomenclature rules of IUPAC-IUB.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa or X.

Further, three-letter codes generally allowed not only for naturally occurring amino acids, but also for other amino acids, such as 2-Aminoisobutyric acid (Aib), Sar (N-methylglycine), α-methyl-glutamic acid, etc. may be used.

Amino acids may be generally classified based on similarity of polarity, charge, solubility, hydrophobicity, hydrophilicity, and/or amphipathic nature of a residue. Accordingly, amino acid substitution may generally occur based on similarity of polarity, charge, solubility, hydrophobicity, hydrophilicity, and/or amphipathic nature of a residue.

For example, among the amino acids having an electrically charged side chain (electrically charged amino acid), positively charged (basic) amino acids comprise arginine, lysine, and histidine; negatively charged (acidic) amino acids comprise glutamic acid and aspartic acid; among the amino acids having an uncharged side chain (uncharged amino acid), nonpolar amino acids comprise glycine, alanine, valine, leucine, isoleucine, methionine, phenylalanine, tryptophan, and proline; polar or hydrophilic amino acids comprise serine, threonine, cysteine, tyrosine, asparagine, and glutamine; and among the nonpolar amino acids, aromatic amino acids comprise phenylalanine, tryptophan, and tyrosine.

In one embodiment, the modified polypeptide having DON degradation activity provided in the present disclosure may comprise a modified polypeptide, in which amino acids at positions corresponding to any one or more positions selected from positions 28, 29, 87, 91, 101, and 152 in SEQ ID NO: 1 are substituted, or may consist of SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33 or SEQ ID NO: 35. In another embodiment, the novel modified polypeptide having DON degradation activity provided in the present disclosure may have at least 80%, 90%, 91%, 92%, 93%, 94%, 95%, 96%, 97%, 98%, 99% or more, or 100% homology or identity to SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33 or SEQ ID NO: 35, or may comprise a sequence having the homology or identity, or may essentially consist of or may consist of the same.

As used herein, the term "homology" or "identity" refers to a degree of relevance between two given amino acid sequences or nucleotide sequences, and may be expressed as a percentage. The terms "homology" and "identity" may often be used interchangeably with each other.

The sequence homology or identity of conserved polynucleotides or polypeptides may be determined by standard alignment algorithms and may be used with a default gap penalty established by the program being used. Substantially, homologous or identical sequences may generally hybridize under moderately or highly stringent conditions to the entire length of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether any two polynucleotide or polypeptide sequences have homology, similarity, or identity may be, for example, determined using a known computer algorithm such as the "FASTA" program using default parameters, as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073. For example, the homology, similarity, or identity may be determined using BLAST or ClustalW of the National Center for Biotechnology Information, but is not limited thereto.

The homology, similarity, or identity of polynucleotides or polypeptides may be, for example, determined by comparing sequence information using, for example, the GAP computer program, such as Needleman et al. (1970), J Mol Biol. 48: 443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. Default parameters for the GAP program may comprise (1) a binary comparison matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have homology, similarity, or identity with each other may be identified by comparing the sequences in a Southern hybridization experiment under stringent conditions as defined, and appropriate hybridization conditions are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

In any one specific embodiment of the above-described specific embodiments, the modified polypeptide may have any one or more altered properties of the following i) to vii), compared to the polypeptide consisting of the amino acid sequence of SEQ ID NO: 1:
i) increased enzyme activity;
ii) increased specific activity;
iii) increased pH stability;
iv) increased storage stability;
v) increased acid resistance;
vi) increased thermal stability; and
vii) altered substrate specificity.

As used herein, "enzymatic activity" or "DON degradation activity" represents at least one catalytic activity. Specifically, it may be the conversion efficiency of an enzyme, which is mainly expressed as kcat/Km, but is not limited thereto.

When the enzyme is completely saturated with substrates, kcat means the rate constant (catalytic constant) that converts substrates into products in unit time by one enzyme, and is also referred to as a turnover number. Km is the substrate concentration when the reaction rate is at half the maximum value (Vmax).

Examples of the ways to express enzymatic activity comprise specific activity (umol of converted substrate x mg ⁻¹ x min ⁻¹) or volumetric activity (umol of converted substrate x mL ⁻¹ x min ⁻¹), etc. However, defining the enzymatic activity is not limited to the description described above, and the enzymatic activity may be defined and evaluated, based on the information known in the art.

As used herein, "enzyme stability" means that enzyme activity is maintained during storage or reaction time. In order to measure such changes in stability, the initial enzyme activity may be measured and compared under defined conditions at time zero (100%) and after a predetermined period of time (x%), and therefore, the level at which the enzyme activity is lost or the enzyme stability may be expressed.

Factors that affect enzyme activity comprise, for example, pH, heat, the presence of other substances (e.g., oxidizing agents, chelating agents), etc.

In one specific embodiment, the modified polypeptide provided in the present disclosure may have an increased enzymatic activity of about 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, 100%, about 110%, about 120%, about 130%, about 140%, about 150%, about 160%, about 170%, about 180%, about 190%, about 200% or more, about 210% or more, about 220% or more, or about 230% or more, as compared to the parent enzyme.

As used herein, the term "specific activity" is the activity of an enzyme per unit weight of protein, and may be expressed as unit/mg. The quantification of protein may be performed using, for example, SDS-PAGE or Bradford assay, etc.

As used herein, the "enzyme stability" means that enzyme activity is maintained during storage or reaction time. In order to measure such changes in stability, the initial enzyme activity may be measured and compared under defined conditions at time zero (100%) and after a predetermined period of time (x%), and therefore, the level at which the enzyme activity is lost or the enzyme stability may be expressed.

Factors that affect enzyme activity comprise, for example, pH, heat, the presence of other substances (e.g., oxidizing agents, chelating agents), etc.

As used herein, the term "thermal stability" means the ability of a protein to function in a specific temperature range. In one embodiment, the polypeptide provided in the present disclosure may have activity in the range of about 20°C to about 120°C, specifically, in the range of about 60°C to about 100°C. In any one embodiment of the above-described embodiments, the polypeptide having DON degradation activity provided in the present disclosure may have activity at 50°C to 80°C, but is not limited thereto.

As used herein, the term "thermal tolerance" means the ability of a protein to function after exposure to a specific temperature, e.g., high heat or cryogenic temperature. For example, proteins having thermal tolerance may not function at a temperature to which they are exposed, but may become functional when returned to an optimal temperature environment.

As used herein, the term "pH stability" means the ability of a protein to function in a specific pH range. In one specific embodiment, the variant provided in the present disclosure may have activity at about pH 2.0 to about pH 12.0, but is not limited thereto.

When a protein maintains its function in a specific pH range, it may be defined as having "pH stability", and may also be defined as having "acid resistance", "alkali resistance", etc., according to the pH range.

In one embodiment of the present disclosure, the variant provided in the present disclosure may have "acid resistance" at about pH 3.0 or lower, but is not limited thereto.

An increase in stability may comprise maintaining high enzymatic activity; increasing the range of pH, temperature, and/or time, etc., at which a protein maintains its function, compared to other enzymes, e.g., a wild-type enzyme, a parent enzyme, and/or other modified polypeptides.

In the present disclosure, the increase in stability may be compared, based on the parent DON degradation enzyme, for example, based on the DON degradation enzyme of SEQ ID NO: 1, but is not limited thereto.

In one embodiment, the modified polypeptide having DON degradation activity provided in the present disclosure may have improvement in one or more properties selected from thermal tolerance, thermal stability, and acid resistance, as compared to the polypeptide of SEQ ID NO: 1.

In any one embodiment of the above-described embodiments, the modified polypeptide may maintain relative activity of at least 1% or more, 2% or more, 3% or more, 4% or more, 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more 29% or more, 30% or more, 31% or more, 32% or more, 33% or more, 34% or more, 35% or more, 37% or more, or 40% or more at 50°C to 80°C, but is not limited thereto.

In any one embodiment of the above-described embodiments, the modified polypeptide may maintain relative activity of at least 1% or more, 3% or more, 5% or more, 8% or more, 10% or more, 15% or more, 20% or more, 25% or more, 30% or more, 35% or more, 40% or more, 45% or more, 50% or more, 55% or more, 60% or more, 65% or more or 70% or more at pH 3 or lower, but is not limited thereto.

In any one embodiment of the above-described embodiments, the modified polypeptide having increased enzymatic activity (improved DON degradation activity), as compared to the polypeptide of SEQ ID NO. 1, among the modified polypeptides, may comprise any one or more substitutions selected from the following substitutions.

| |
|---|
| F28I |
| K101F, K101W, K101P, K101T or K101E |
| D152G |
| M29L |
| A87I |
| G91S |
| F28I+M29L |
| F28I+A87I |
| F28I+G91S |
| F28I+K101F(K101W, K101P, K101T or K101E) |
| F28I+D152G |
| M29L+A87I |
| M29L+G91S |
| M29L+K101F(K101W, K101P, K101T or K101E) |
| M29L+D152G |
| M29L+D152G |
| A87I+ G91S |
| A87I+ K101F(K101W, K101P, K101T or K101E) |
| A87I+ D152G |
| G91S+ K101F(K101W, K101P, K101T or K101E) |
| G91S+ D152G |
| K101F(K101W, K101P, K101T or K101E)+ D152G |
| F28I+ M29L+ A87I |
| F28I+ M29L+ K101F(K101W, K101P, K101T or K101E) |
| F28I+ M29L+ D152G |
| F28I+ A87I+ G91S |
| F28I+ A87I+ K101F(K101W, K101P, K101T or K101E) |
| F28I+ A87I+ D152G |
| F28I+ G91S+ K101F(K101W, K101P, K101T or K101E) |
| F28I+ G91S+ D152G |
| F28I+ K101F(K101W, K101P, K101T or K101E)+ D152G |
| M29L+ A87I+ G91S |
| M29L+ A87I+ K101F(K101W, K101P, K101T or K101E) |
| M29L+ A87I+ D152G |
| F281+G91S+ K101F(K101W, K101P, K101T or K101E) |
| M29L+ G91S+ D152G |
| M29L+ K101F(K101W, K101P, K101T or K101E)+ D152G |
| A87I+ G91S+ K101F(K101W, K101P, K101T or K101E) |
| A87I+ G91S+ D152G |
| A87I+ K101F(K101W, K101P, K101T or K101E)+ D152G |
| G91S+ K101F(K101W, K101P, K101T or K101E)+ D152G |
| F28I+ M29L+ A87I+ G91S |
| F28I+ M29L+ A87I+ K101F(K101W, K101P, K101T or K101E) |
| F28I+ M29L+ A87I+ D152G |
| F28I+ M29L+ G91S+ K101F(K101W, K101P, K101T or K101E) |
| F28I+ M29L+ G91S+ D152G |
| F28I+ M29L+ K101F(K101W, K101P, K101T or K101E)+ D152G |
| F28I+ A87I+ G91S+ K101F(K101W, K101P, K101T or K101E) |
| F28I+ A87I+ G91S+ D152G |
| F28I+ A87I+ K101F(K101W, K101P, K101T or K101E)+ D152G |
| F28I+ G91S+ K101F(K101W, K101P, K101T or K101E)+ D152G |
| M29L+ A87I+ G91S+ K101F(K101W, K101P, K101T or K101E) |
| M29L+ A87I+ G91S+ D152G |
| M29L+ A87I+ K101F(K101W, K101P, K101T or K101E)+ D152G |
| M29L+ G91S+ K101F(K101W, K101P, K101T or K101E)+ D152G |
| A87I+ G91S+ K101F(K101W, K101P, K101T or K101E)+ D152G |
| F28I+ M29L+ A87I+ G91S+ K101F(K101W, K101P, K101T or K101E) |
| F28I+ M29L+ A87I+ G91S+ D152G |
| F28I+ M29L+ A87I+ K101F(K101W, K101P, K101T or K101E)+ D152G |
| F28I+ M29L+ G91S+ K101F(K101W, K101P, K101T or K101E)+ D152G |
| F28I+ A87I+ G91S+ K101F(K101W, K101P, K101T or K101E)+ D152G |
| M29L+ A87I+ G91S+ K101F+ D152G |
| F281+ M29L+ A871+ G91S+ K101F(K101W, K101P, K101T or K101E)+ D152G |

In any one embodiment of the above-described embodiments, the modified polypeptide having improved thermal tolerance, as compared to the polypeptide of SEQ ID NO. 1, among the modified polypeptides, may comprise any one or more substitutions selected from the following substitutions.

| |
|---|
| F28I |
| K101F, K101G, K101V, K101I, K101M, K101S, K101T, K101C, K101Y, K101N, K101Q, K101D, K101R or K101H |
| M29L |
| A87I |
| G91S |
| F28I+M29L |
| F28I+A87I |
| F28I+G91S |
| F28I+K101F(K101G, K101V, K101I, K101M, K101S, K101T, K101C, K101Y, K101N, K101Q, K101D, K101R or K101H) |
| M29L+A87I |
| M29L+G91S |
| M29L+K101F(K101G, K101V, K101I, K101M, K101S, K101T, K101C, K101Y, K101N, K101Q, K101D, K101R or K101H) |
| A87I+ G91S |
| A87I+ K101F(K101G, K101V, K101I, K101M, K101S, K101T, K101C, K101Y, K101N, K101Q, K101D, K101R or K101H) |
| G91S+ K101F(K101G, K101V, K101I, K101M, K101S, K101T, K101C, K101Y, K101N, K101Q, K101D, K101R or K101H) |
| F28I+ M29L+ A87I |
| F28I+ M29L+ K101F(K101G, K101V, K101I, K101M, K101S, K101T, K101C, K101Y, K101N, K101Q, K101D, K101R or |
| K101H) |
| F28I+ A87I+ G91S |
| F28I+ A87I+ K101F(K101G, K101V, K101I, K101M, K101S, K101T, K101C, K101Y, K101N, K101Q, K101D, K101R or K101H) |
| F28I+ G91S+ K101F(K101G, K101V, K101I, K101M, K101S, K101T, K101C, K101Y, K101N, K101Q, K101D, K101R or K101H) |
| M29L+ A87I+ G91S |
| M29L+ A87I+ K101F(K101G, K101V, K101I, K101M, K101S, K101T, K101C, K101Y, K101N, K101Q, K101D, K101R or K101H) |
| F28I+G91S+ K101F(K101G, K101V, K101I, K101M, K101S, K101T, K101C, K101Y, K101N, K101Q, K101D, K101R or K101H) |
| A87I+ G91S+ K101F(K101G, K101V, K101I, K101M, K101S, K101T, K101C, K101Y, K101N, K101Q, K101D, K101R or K101H) |
| F28I+ M29L+ A87I+ G91S |
| F28I+ M29L+ A87I+ K101F(K101G, K101V, K101I, K101M, K101S, K101T, K101C, K101Y, K101N, K101Q, K101D, K101R or K101H) |
| F28I+ M29L+ G91S+ K101F(K101G, K101V, K101I, K101M, K101S, K101T, K101C, K101Y, K101N, K101Q, K101D, K101R or K101H) |
| F28I+ A87I+ G91S+ K101F(K101G, K101V, K101I, K101M, K101S, K101T, K101C, K101Y, K101N, K101Q, K101D, K101R or K101H) |
| M29L+ A87I+ G91S+ K101F(K101G, K101V, K101I, K101M, K101S, K101T, K101C, K101Y, K101N, K101Q, K101D, K101R or K101H) |
| F28I+ M29L+ A87I+ G91S+ K101F(K101G, K101V, K101I, K101M, K101S, K101T, K101C, K101Y, K101N, K101Q, K101D, K101R or K101H) |

In any one embodiment of the above-described embodiments, the modified polypeptide having improved acid resistance, as compared to the polypeptide of SEQ ID NO. 1, among the modified polypeptides, may comprise any one or more substitutions selected from the following substitutions.

| |
|---|
| F28I |
| K101F, K101I, K101M, K101T, K101C, K101Y, K101Q, K101E, K101RorK101H |
| D152G |
| M29L |
| A87I |
| G91S |
| F28I+M29L |
| F28I+A87I |
| F28I+G91S |
| F28I+K101F(K1011, K101M, K101T, K101C, K101Y, K101Q, K101E, K101R or K101H) |
| F28I+D152G |
| M29L+A87I |
| M29L+G91S |
| M29L+K101F(K101I, K101M, K101T, K101C, K101Y, K101Q, K101E, K101R or K101H) |
| M29L+D152G |
| M29L+D152G |
| A87I+ G91S |
| A87I+ K101F(K101I, K101M, K101T, K101C, K101Y, K101Q, |
| K101E, K101R or K101H) |
| A87I+ D152G |
| G91S+ K101F(K101I, K101M, K101T, K101C, K101Y, K101Q, K101E, K101R or K101H) |
| G91S+ D152G |
| K101F(K101I, K101M, K101T, K101C, K101Y, K101Q, K101E, K101R or K101H)+ D152G |
| F28I+ M29L+ A87I |
| F28I+ M29L+ K101F(K101I, K101M, K101T, K101C, K101Y, K101Q, K101E, K101R or K101H) |
| F28I+ M29L+ D152G |
| F28I+ A87I+ G91S |
| F28I+ A87I+ K101F(K101I, K101M, K101T, K101C, K101Y, K101Q, K101E, K101R or K101H) |
| F28I+ A87I+ D152G |
| F28I+ G91S+ K101F(K101I, K101M, K101T, K101C, K101Y, K101Q, K101E, K101R or K101H) |
| F28I+ G91S+ D152G |
| F28I+ K101F(K101I, K101M, K101T, K101C, K101Y, K101Q, K101E, K101R or K101H)+ D152G |
| M29L+ A87I+ G91S |
| M29L+ A87I+ K101F(K101I, K101M, K101T, K101C, K101Y, K101Q, K101E, K101R or K101H) |
| M29L+ A87I+ D152G |
| F281+G91S+ K101F(K101I, K101M, K101T, K101C, K101Y, K101Q, K101E, K101R or K101H) |
| M29L+ G91S+ D152G |
| M29L+ K101F(K101I, K101M, K101T, K101C, K101Y, K101Q, K101E, K101R or K101H)+ D152G |
| A87I+ G91S+ K101F(K101I, K101M, K101T, K101C, K101Y, |
| K101Q, K101E, K101R or K101H) |
| A87I+ G91S+ D152G |
| A87I+ K101F(K101I, K101M, K101T, K101C, K101Y, K101Q, K101E, K101R or K101H)+ D152G |
| G91S+ K101F(K101I, K101M, K101T, K101C, K101Y, K101Q, K101E, K101R or K101H)+ D152G |
| F28I+ M29L+ A87I+ G91S |
| F28I+ M29L+ A87I+ K101F(K101I, K101M, K101T, K101C, K101Y, K101Q, K101 E, K101R or K101H) |
| F28I+ M29L+ A87I+ D152G |
| F28I+ M29L+ G91S+ K101F(K101I, K101M, K101T, K101C, K101Y, K101Q, K101E, K101R or K101H) |
| F28I+ M29L+ G91S+ D152G |
| F28I+ M29L+ K101F(K101I, K101M, K101T, K101C, K101Y, K101Q, K101E, K101R or K101H)+ D152G |
| F28I+ A87I+ G91S+ K101F(K101I, K101M, K101T, K101C, K101Y, K101Q, K101E, K101R or K101H) |
| F28I+ A87I+ G91S+ D152G |
| F28I+ A87I+ K101F(K101I, K101M, K101T, K101C, K101Y, K101Q, K101E, K101R or K101H)+ D152G |
| F28I+ G91S+ K101F(K101I, K101M, K101T, K101C, K101Y, K101Q, K101E, K101R or K101H)+ D152G |
| M29L+ A87I+ G91S+ K101F(K101I, K101M, K101T, K101C, K101Y, K101Q, K101E, K101R or K101H) |
| M29L+ A87I+ G91S+ D152G |
| M29L+ A87I+ K101F(K101I, K101M, K101T, K101C, K101Y, K101Q, K101E, K101R or K101H)+ D152G |
| M29L+ G91S+ K101F(K101I, K101M, K101T, K101C, K101Y, K101Q, K101E, K101R or K101H)+ D152G |
| A87I+ G91S+ K101F(K101I, K101M, K101T, K101C, K101Y, |
| K101Q, K101E, K101R or K101H)+ D152G |
| F28I+ M29L+ A87I+ G91S+ K101F(K101I, K101M, K101T, K101C, K101Y, K101Q, K101E, K101R or K101H) |
| F28I+ M29L+ A87I+ G91S+ D152G |
| F28I+ M29L+ A87I+ K101F(K101I, K101M, K101T, K101C, K101Y, K101Q, K101E, K101R or K101H)+ D152G |
| F28I+ M29L+ G91S+ K101F(K101I, K101M, K101T, K101C, K101Y, K101Q, K101E, K101R or K101H)+ D152G |
| F28I+ A87I+ G91S+ K101F(K101I, K101M, K101T, K101C, K101Y, K101Q, K101E, K101R or K101H)+ D152G |
| M29L+ A87I+ G91S+ K101F(K101I, K101M, K101T, K101C, K101Y, K101Q, K101E, K101R or K101H)+ D152G |
| F281+ M29L+ A871+ G91S+ K101F(K101I, K101M, K101T, K101C, K101Y, K101Q, K101E, K101R or K101H)+ D152G |

An increase in stability may comprise maintaining high enzymatic activity; increasing the range of pH, temperature and/or time, etc., at which a protein maintains its function, compared to other enzymes, e.g., a wild-type enzyme, a parent enzyme, and/or other variants.

A decrease in stability may comprise maintaining low enzymatic activity; decreasing the range of pH, temperature and/or time, etc., at which a protein maintains its function, compared to other enzymes, e.g., a wild-type enzyme, a parent enzyme, and/or other variants.

In any one embodiment of the above-described embodiments, the properties possessed by the modified polypeptide having DON degradation activity, which is provided in the present disclosure, may be suitable for or have improved activity for application in various industrial fields, including feeds, baking, pulp bleaching, etc.

Another aspect of the present disclosure provides a polynucleotide encoding the modified polypeptide having DON degradation activity of the present disclosure.

As used herein, the term "polynucleotide", which is a long chain polymer of nucleotides formed by linking nucleotide monomers via covalent bonds, refers to a DNA or RNA strand having a predetermined length or more.

The polynucleotide encoding the modified polypeptide having DON degradation activity of the present disclosure may comprise any polynucleotide without limitation, as long as it is a polynucleotide encoding the modified polypeptide; the polypeptide of SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33, or SEQ ID NO: 35 or the polypeptide having activity corresponding thereto.

The polynucleotide encoding the modified polypeptide having DON degradation activity of the present disclosure may undergo various modifications in the coding region within the scope that does not change the amino acid sequence of the polypeptide, due to codon degeneracy or in consideration of the codons preferred in an organism in which the polypeptide is to be expressed.

For example, the polynucleotide encoding the modified polypeptide having DON degradation activity of the present disclosure may have an amino acid sequence of SEQ ID NO: SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33, or SEQ ID NO: 35, or a polynucleotide encoding a polypeptide having homology or identity thereto.

In one embodiment, the polynucleotide encoding the modified polypeptide having DON degradation activity of the present disclosure may have or comprise a nucleotide sequence having at least 50%, 60%, 70%, 75%, 80%, 85%, or 90% or more, and less than 100% homology or identity to a polynucleotide sequence of SEQ ID NO: 4, SEQ ID NO: 6, SEQ ID NO: 8, SEQ ID NO: 10, SEQ ID NO: 12, SEQ ID NO: 14, SEQ ID NO: 32, SEQ ID NO: 34, or SEQ ID NO: 36. In another embodiment, the polynucleotide encoding the modified polypeptide having DON degradation activity of the present disclosure may consist of or essentially consist of a nucleotide sequence having at least 70%, 75%, 80%, 85%, 90%, 95%, 96%, 97%, 98%, or 99% or more homology or identity to the sequence of SEQ ID NO: 4, SEQ ID NO: 6, SEQ ID NO: 8, SEQ ID NO: 10, SEQ ID NO: 12, SEQ ID NO: 14, SEQ ID NO: 32, SEQ ID NO: 34, or SEQ ID NO: 36, but is not limited thereto. Further, the polynucleotide of the present disclosure may include a probe that may be prepared from a known gene sequence, e.g., any sequence capable of hybridizing with a sequence complementary to all or part of the polynucleotide sequence of the present disclosure under stringent conditions without limitation.

The "stringent conditions" refer to conditions under which specific hybridization between polynucleotides is allowed. Such conditions are specifically described in the literature (see J. Sambrook et al., Molecular Cloning, A Laboratory Manual, 2nd Edition, Cold Spring Harbor Laboratory Press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having high homology or identity, polynucleotides having 70% or more, 75% or more, 80% or more, 85% or more, 90% or more, 95% or more, 96% or more, 97% or more, 98% or more, or 99% or more homology or identity are hybridized with each other, and polynucleotides having homology or identity lower than the above are not hybridized with each other, or washing conditions of the common Southern hybridization, i.e., washing once, specifically, twice or three times at a salt concentration and a temperature corresponding to 60°C, 1×SSC, 0.1% SDS, specifically, 60°C, 0.1×SSC, 0.1% SDS, and more specifically 68°C, 0.1×SSC, 0.1% SDS.

Hybridization requires that two nucleic acids comprise complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may comprise isolated nucleotide fragments complementary to the entire sequence as well as nucleic acid sequences substantially similar thereto.

Specifically, polynucleotides having homology or identity to the polynucleotide of the present disclosure may be detected using the hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (e.g., J. Sambrook et al., supra).

Still another aspect of the present disclosure provides a vector comprising the polynucleotide encoding the modified polypeptide having DON degradation activity of the present disclosure. The modified polypeptide and polynucleotide are as described in other aspects.

As used herein, the term "vector" refers to a DNA construct comprising the nucleotide sequence of the polynucleotide encoding the desired polypeptide operably linked to a suitable expression regulatory region (expression regulatory sequence) so as to express the desired polypeptide in a suitable host cell. The expression regulatory region may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into the genome thereof.

For example, the polynucleotide encoding the desired protein may be replaced with a mutated polynucleotide in the chromosome through a vector for intracellular chromosomal insertion. The insertion of the polynucleotide into the chromosome may be performed by any method known in the art, e.g., by homologous recombination, but is not limited thereto. The vector may further comprise a selection marker to confirm the insertion into the chromosome. The selection marker is for selecting the cells transformed with the vector, i.e., for confirming whether the desired nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or exhibit different phenotypes under the environment treated with the selective agent, and thus the transformed cells may be selected.

The vector used in the present disclosure is not particularly limited, and any vector known in the art may be used.

Examples of the vectors commonly used in prokaryotic cells may comprise, as phage vectors or cosmid vectors, pWE15, M13, MBL3, MBL4, IXII, ASHII, APII, t10, t11, Charon4A, and Charon21A; and as plasmid vectors, those based on pBR, pUC, pBluescriptll, pGEM, pTZ, pCL and pET, etc. Specifically, pDZ, pACYC177, pACYC184, pCL, pECCG117, pUC19, pBR322, pMW118, pCC1BAC, pDCM2 vector, etc. may be used.

Examples of the vectors used in eukaryotic cells may comprise, as yeast expression vectors, integrative yeast plasmids (YIp) and extrachromosomal plasmid vectors. The extrachromosomal plasmid vectors may comprise episomal yeast plasmids (YEp), replicative yeast plasmids (YRp), and yeast centromeric plasmids (YCp). Further, artificial yeast chromosomes (YACs) may also be used as the vector of the present disclosure. Specific examples of the available vectors may include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NTA, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405, and pYJ406, but are not limited thereto.

As used herein, the term "transformation" means that a vector comprising a polynucleotide encoding a target protein is introduced into a host cell or a microorganism so that the protein encoded by the polynucleotide may be expressed in the host cell. The transformed polynucleotide may be located regardless of the position, either by being inserted into the chromosome of the host cell or located outside the chromosome as long as it may be expressed in the host cell. Further, the polynucleotide includes DNA and RNA encoding a desired protein. The polynucleotide may be introduced in any form as long as it may be introduced into a host cell and expressed. For example, the polynucleotide may be introduced into a host cell in the form of an expression cassette, which is a gene construct comprising all elements required for self-expression. The expression cassette may usually include a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, the term "operably linked" means that the gene sequence is functionally linked to a promoter sequence that initiates and mediates transcription of the polynucleotide encoding the desired polypeptide of the present disclosure.

The method of transforming the vector of the present disclosure includes any method of introducing the nucleic acid into a cell, and may be performed by selecting a suitable standard technique known in the art according to the host cell. For example, the transformation method may include electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

Still another aspect of the present disclosure provides a microorganism comprising any one or more of the modified polypeptide having DON degradation activity of the present disclosure; and the polynucleotide encoding the modified polypeptide.

The microorganism may comprise the above-described modified polypeptide, the polynucleotide encoding the same, a nucleic construct and/or vector comprising the polynucleotide. The nucleic acid construct or vector may be integrated into the chromosome, or may remain as a self-replicating extrachromosomal vector.

The microorganism of the present disclosure may comprise any microorganism without limitation as long as it is able to express the modified polypeptide having DON degradation activity of the present disclosure. For example, the microorganism may comprise any cell useful in the recombinant production of the modified polypeptide having DON degradation activity of the present disclosure. For example, the microorganism may be a prokaryotic or eukaryotic cell. For another example, examples of the microorganism may comprise fungi and bacteria.

In one embodiment, the microorganism of the present disclosure may be a microorganism of the genus *Escherichia,* and may be *Escherichia coli* (*E. coli*), *Escherichia albertii, Escherichia fergusonii, Escherichia hermannii, Escherichia vulneris,* or *Escherichia blattae.* In any one embodiment of the above-described embodiments, the microorganism may be *Escherichia coli,* but is not limited thereto.

Still another aspect of the present disclosure provides a composition for degrading deoxynivalenol (DON), the composition comprising any one or more of the modified polypeptide having DON degradation activity of the present disclosure; and the microorganism expressing the modified polypeptide.

Still another aspect of the present disclosure provides a method of degrading deoxynivalenol (DON), the method comprising the step of reacting DON with any one or more of the modified polypeptide having DON degradation activity of the present disclosure; and the microorganism expressing the modified polypeptide.

The modified polypeptide having DON degradation activity of the present disclosure and/or the microorganism expressing the modified polypeptide may be used in degrading substances comprising DON.

In one specific embodiment, the polypeptide of the present disclosure may be used in degradation of DON toxin, detoxification of DON, conversion of DON (deoxynivalenol) to iso-DON (iso-deoxynivalenol), and/or the induction of degradation through the conversion of iso-DON.

Further, in another additional embodiment of the present disclosure, the polypeptide of the present disclosure may be used in detoxification of foods contaminated with DON, removal of contamination (decontaminating), or detoxification (e.g., degradation).

In one embodiment, the composition for degrading DON provided in the present disclosure may further comprise a naturally occurring substance or non-naturally occurring substance, in addition to the modified polypeptide of the present disclosure.

In one embodiment, the composition for degrading DON provided in the present disclosure may further comprise any components suitable for application in various industrial fields such as animal feeds, baking, biomass saccharification, pulp bleaching, etc.

Examples of the substances that may be added comprise stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, stabilizers, diluents, lubricants, preservatives, etc., but are not limited thereto.

Still another aspect of the present disclosure provides a feed additive composition comprising any one or more of the modified polypeptide having DON degradation activity of the present disclosure; and the microorganism expressing the modified polypeptide.

Still another aspect of the present disclosure provides a method of preparing a feed product, the method comprising the step of mixing a feed component with the feed additive composition comprising any one or more of the modified polypeptide having DON degradation activity of the present disclosure; and the microorganism expressing the modified polypeptide.

The modified polypeptide having DON degradation activity of the present disclosure and/or the microorganism expressing the modified polypeptide may be used in any one of the following applications:
a) an additive in animal feedstuffs; and/or
b) a feed supplement for animals; and/or
c) breakdown of grain-based materials (e.g., this may be whole grain or part of grain).

The feed composition of the present disclosure may refer to any natural or artificial diet, a single meal, etc., or a component of the single meal, which is eaten, ingested, and digested by an animal, or which is suitable therefor, and the feed composition may be prepared in various forms known in the art.

In one embodiment, the feed product may be a grain-based material (including whole or part of grains, or malted grains, e.g., wheat, barley, corn, oats, rye, rice, sorghum, etc.).

Still another aspect of the present disclosure provides a method of preparing the modified polypeptide having DON degradation activity, the method comprising the steps of:
culturing the microorganism comprising any one or more of the modified polypeptide having DON degradation activity of the present disclosure; and the polynucleotide encoding the modified polypeptide; and
recovering the modified polypeptide having DON degradation activity of the present disclosure, which is expressed in the culturing step.

The culturing may comprise the step of culturing the microorganism in a culture medium.

As used herein, the term "culturing" means that the host cell is grown under appropriately controlled environmental conditions. The culturing process of the present disclosure may be performed in a suitable medium and culture conditions known in the art. Such a culturing process may be easily adjusted for use by those skilled in the art according to the strain to be selected. Specifically, the culturing may be a batch culture, a continuous culture, or a fed-batch culture, but is not limited thereto.

As used herein, the term "medium" refers to a mixture of materials that comprise nutrient materials required for culturing the host cell as a main ingredient, and it supplies nutrient materials and growth factors, along with water that is essential for survival and growth. Specifically, the medium and other culture conditions used for culturing the host cell of the present disclosure may include any medium commonly used for culturing host cells without any particular limitation. However, the host cell of the present disclosure may be cultured under aerobic conditions in a common medium comprising appropriate carbon sources, nitrogen sources, phosphorus sources, inorganic compounds, amino acids, and/or vitamins, while adjusting temperature, pH, etc.

In the present disclosure, the carbon source may include carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; amino acids, such as glutamic acid, methionine, lysine, etc. Further, natural organic nutrients such as starch hydrolysate, molasses, blackstrap molasses, rice bran, cassava, sugar cane molasses, and corn steep liquor, etc. may be used. Specifically, carbohydrates such as glucose and sterilized pretreated molasses (i.e., molasses converted to reducing sugar) may be used, and in addition, various other carbon sources may be used in an appropriate amount without limitation. These carbon sources may be used alone or in a combination of two or more thereof, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extract, yeast extract, malt extract, corn steep liquor, casein hydrolysate, fish or decomposition product thereof, defatted soybean cake or decomposition product thereof, etc. These nitrogen sources may be used alone or in a combination of two or more thereof, but are not limited thereto.

The phosphorus source may include monopotassium phosphate, dipotassium phosphate, or corresponding sodium-containing salts, etc. Examples of the inorganic compounds may include sodium chloride, calcium chloride, iron chloride, magnesium sulfate, iron sulfate, manganese sulfate, calcium carbonate, etc. In addition, amino acids, vitamins, and/or appropriate precursors may be comprised. These components or precursors may be added to a medium in a batch or continuous manner, but are not limited thereto.

Further, the pH of the medium may be adjusted by adding a compound such as ammonium hydroxide, potassium hydroxide, ammonia, phosphoric acid, sulfuric acid, etc. during the culturing of the host cell in an appropriate manner. In addition, foam formation may be prevented during the culturing by using an antifoaming agent such as fatty acid polyglycol ester. Further, oxygen or oxygen-containing gas may be injected into the medium in order to maintain aerobic conditions of the medium; or no gas may be injected, or nitrogen, hydrogen, or carbon dioxide gas may be injected in order to maintain anaerobic or microaerobic conditions, but this is not limited thereto.

The temperature of the medium may be 20°C to 55°C, specifically, 25°C to 40°C, but is not limited thereto. The culturing may be continued until the desired amount of a useful material is obtained, and may be specifically carried out for 24 hours to 196 hours, but is not limited thereto.

In one embodiment, the modified polypeptide having DON degradation activity which is expressed in the culturing step may be recovered using methods known in the art to which the present disclosure pertains. For example, the modified polypeptide may be recovered from a medium by common procedures including, but not limited to, collection, centrifugation, filtration, extraction, spray-drying, evaporation, or precipitation.

The recovering method may be collecting the polypeptide using the method of culturing the microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, sonication, ultrafiltration, dialysis, various kinds of chromatographies such as molecular sieve chromatography (gel filtration), adsorption chromatography, ion exchange chromatography, affinity chromatography, etc., HPLC and a combination thereof may be used, and the modified polypeptide of the present disclosure may be recovered from the medium or the host cell using suitable methods known in the art.

In another embodiment, the modified polypeptide expressed by the host cell in the culturing step may not be recovered. In the embodiment, the host cell itself expressing the modified polypeptide may be used as a source of the modified polypeptide.

Still another aspect of the present disclosure provides a use of the modified polypeptide of the present disclosure for the degradation of DON; and a use for the degradation of DON by one or more microorganisms expressing the modified polypeptide.

The definitions of the terms are as described above.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1: Preparation of GhSPG-216M2 variants

### Example 1-1. Preparation of GhSPG-216M2

A variant (GhSPG-216; SEQ ID NO: 21) in which 35 mutations were introduced into a *Gossypium harknessii-derived* hypothetical protein sequence (SEQ ID NO: 23) was synthesized by Cosmo Genetech, and then a polynucleotide (SEQ ID NO: 2) encoding a variant (hereinafter, referred to as GhSPG-216M2, SEQ ID NO: 1) in which two mutations were further added was cloned into a pET vector (Novagen) to prepare an expression vector.

### Example 1-2. Preparation of GhSPG-216M2 point mutation and combination variants

To improve activity, thermal stability, and acid resistance of GhSPG-216M2, mutation sites were selected and primers were designed to prepare 9 kinds of point mutation and combination variants (hereinafter, referred to as 216M3, 216M4, 216M5, 216M6, 216M7, 216M8, 216M9, 216M10, and 216M11, SEQ ID NOS: 3, 5, 7, 9, 11, 13, 31, 33, and 35). The mutation sites based on the amino acid sequence of SEQ ID NO: 1, post-mutation amino acids, and primer sequences for preparing the variants are listed in order in Table 1 below.

**[Table 1]**

| Name of variant | Mutation | | SE Q ID NO. | Primer sequence (5' → 3') |
|---|---|---|---|---|
| 216M3 | F281 | Forward | 15 | GGCCACTAAGGGTTATattATGCAACAGACAATGTT TCGCAT |
| | | Reverse | 16 | CATaatATAACCCTTAGTGGCCTCGTCAGGTGGAG TTTGCAAG |
| 216M4 | K101F | Forward | 17 | ATttttTGGGGTACCGAGAGCGACCCGGAGTTTAAA GGTTAC |
| | | Reverse | 18 | GCTCTCGGTACCCCAaaaATGGGTCAATTCGATTG TCGC |
| 216M5 | D152G | Forward | 19 | AGAATTTGTGAAAAAGCCGggcGACGGCTATGCGT TTATTAAAGACCC |
| | | Reverse | 20 | gccCGGCTTTTTCACAAATTCTACCCCAAGACGTT CGAATCTCTCACAC |
| 216M9 | M29L | Forward | 25 | GGCCACTAAGGGTTATTTTctgCAACAGACAATGTT TCGCATTAAAGAC |
| | | Reverse | 26 | cagAAAATAACCCTTAGTGGCCTCGTCAGGTGGAG TTTGCAAGCC |
| 216M10 | A871 | Forward | 27 | AGCGGACCattTGGACATTTGGCAGACCAGCGACA ATC |
| | | Reverse | 28 | AAATGTCCAaatGGTCCGCTCTATTGGATCACTTG GCGC |
| 216M11 | G91S | Forward | 29 | CCTGGACATTTagcAGACCAGCGACAATCGAATTG ACCC |
| | | Reverse | 30 | TGGTCTgctAAATGTCCAGGCGGTCCGCTCTATTG GATC |
| 216M6 | F281/K1 01F | SEQ ID NOS: 15, 16, 17, 18 applied | | |
| 216M7 | F281/D1 52G | SEQ ID NOS: 15, 16, 19, 20 applied | | |
| 216M8 | F281/K1 01F/D15 2G | SEQ ID NOS: 15, 16, 17, 18, 19, 20 applied | | |

In detail, 9 kinds of point mutation and combination variants of GhSPG-216M2 were prepared by PCR using the polynucleotide encoding GhSPG-216M2 (SEQ ID NO: 1) prepared in Example 1-1 which was cloned into the pET vector, as a template, primers (SEQ ID NOS: 15, 16, 17, 18, 19, 20, 25, 26, 27, 28, 29, 30 and combinations thereof in Table 1), and a PCR premix (iNtRON, cat no. 25185). PCR was performed using an Eppendorf Mastercycler Nexus GX2, and the reaction conditions are as follows:
Initial denaturation - 94°C, 2 min
Denaturation - 94°C, 20 sec
Annealing - 50°C, 10 sec
Extension - 72°C, 8 min (15 cycles from denaturation to extension)
Final Extension - 72°C, 5 min

The prepared truncated variants were ligated using a QuickChange Site-Directed Mutagenesis kit (Agilent, Cat# 200518), and then transformed into the E. *coli* Dh5α strain to identify the presence of sequence mutations through sequencing.

### Example 2: Evaluation of properties of GhSPG-216M2 variants

### Example 2-1. Comparative evaluation of activity of GhSPG-216M2 and variants

The expression vectors of GbSPG-216M2 and 9 kinds of variants thereof prepared in Example 1-1 were each transformed into *E. coli* BL21 (DE3), which were inoculated into a sterile LB medium (BD Difco), and pre-cultured at 37°C, 200 rpm for 16 hours. Thereafter, 1/100 of the medium volume was inoculated into a flask comprising sterile LB medium, and cultured at 37°C, 200 rpm until absorbance (OD₆₀₀) was between 0.4 to 0.5, and then isopropyl β-D-1-thiogalactopyranoside (IPTG) was added to a final concentration of 1 mM, and cultured for an additional 16 hours, and then the cells were collected by centrifugation. The collected cells were added and resuspended in 20 ml of a lysis buffer (50 mM Tris-HCl pH 8.0, 100 mM NaCl, 10 mM imidazole), and a crude enzyme solution was obtained through sonication and centrifugation. The crude enzyme solution was applied and adsorbed onto Ni-NTA resin (Qiagen, Cat no. 30230), and then the enzyme was purified by sequentially applying a washing buffer (only 20 mM imidazole concentration in the lysis buffer composition) and an elution buffer (only 250 mM imidazole concentration in the lysis buffer composition).

Protein concentrations were determined by mixing 5 µl of the diluted enzyme solution + 250 µl of Bradford solution (Quick Start^{™} Bradford 1x Dye Reagent, #5000205), and measuring absorbance at 595 nm.

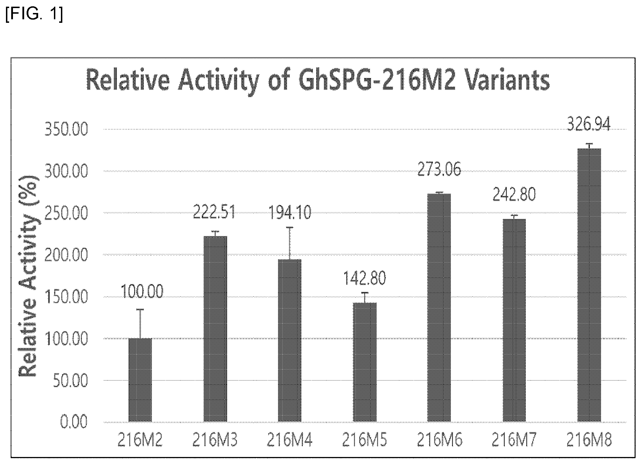

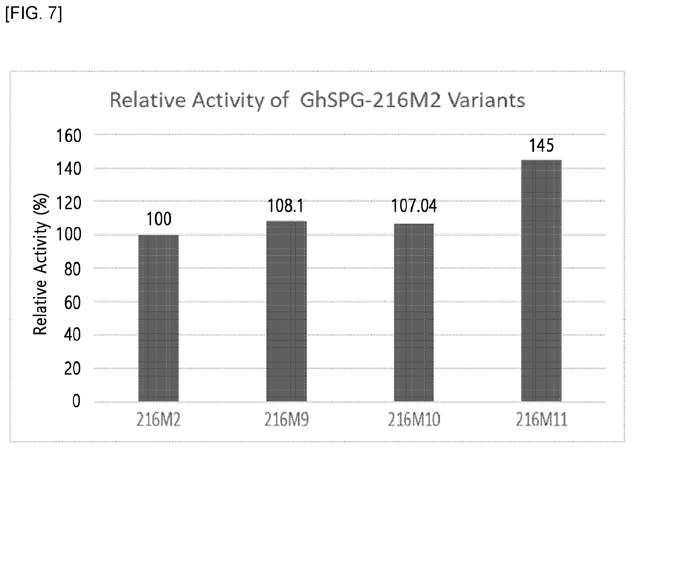

To analyze the activity of purified GhSPG-216M2 and 9 kinds of GhSPG-216M2 variants for DON, DON (CAS 51481-10-8, Deoxynivalenol) dissolved in distilled water and the purified enzyme reaction solution (25 mM Tris HCl, 150 mM NaCl, pH 7.4) were prepared, and after treating with 500 µM of NiCl₂ as a co-factor, the reaction was allowed at 50°C for 24 hours and then stopped by leaving the same at 100°C for 5 minutes. The residual amount of DON in the reaction product was measured using a high performance liquid chromatography-ultraviolet (HPLC-UV) detector, and the relative activities of the variants were measured using the ratio of the total degraded DON, and the results are as shown in FIGS. 1 and 7.

As a result, as shown in FIGS. 1 and 7, it was confirmed that when the enzyme activity was expressed as a relative ratio, the activities of 9 kinds of GhSPG-216M2 variants (216M3, 216M4, 216M5, 216M6, 216M7, 216M8, 216M9, 216M10, 216M11) were improved by 7% to 227%, as compared to the template GhSPG-216M2.

### Example 2-2. Comparative evaluation of thermal stability of GhSPG-216M2 and variants

The GhSPG-216M2 and 6 kinds of variants thereof, 216M3, 216M4, 216M5, 216M6, 216M7, and 216M8, prepared in Example 1, were each transformed into E. *coli* BL21 (DE3), and the enzymes were expressed, purified, and reacted using the same method presented in Example 2-1, and then the reaction products were analyzed using HPLC-UV.

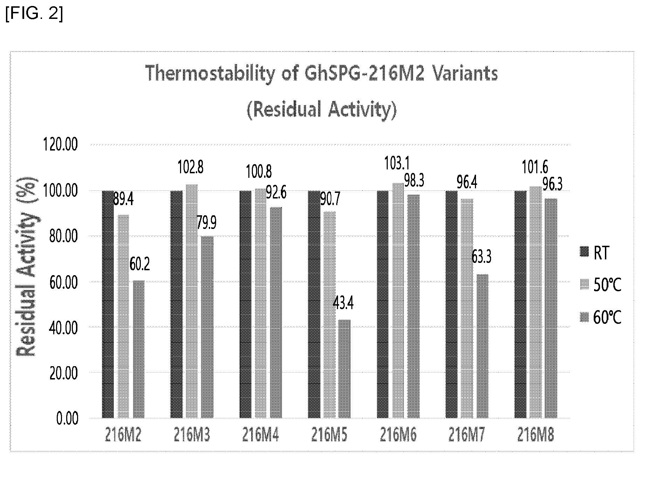

To evaluate thermal stability (residual activity) of GhSPG-216M2 and 6 kinds of variants thereof, 50 µL of each purified enzyme was incubated at room temperature, 50°C, and 60°C for 10 minutes, and the enzyme solutions before and after heat treatment were used to obtain the residual amount of DON after the DON degradation, the decomposed ratio, and the residual activity, which are shown in FIG. 2.

As a result, when left at 50°C for 10 minutes, the residual activities of GhSPG-216M2, 216M3, 216M4, 216M5, 216M6, 216M7, and 216M8, relative to those before heat treatment, were 89.4%, 102.8%, 100.8%, 90.7%, 103.1%, 96.5%, and 101.6%, respectively, indicating that residual activities of the variants were all improved. When left at 60°C for 10 minutes, the residual activities of GhSPG-216M2, 216M3, 216M4, 216M6, 216M7, and 216M8, relative to those before heat treatment, were 60.3%, 79.9%, 92.6%, 98.3%, 63.3%, and 96.3%, respectively, indicating that residual activities of the variants were all improved.

### Example 2-3. Comparative evaluation of acid resistance of GhSPG-216 M2 and variants

GhSPG-216M2 and 6 kinds of variants 216M3, 216M4, 216M5, 216M6, 216M7, and 216M8 prepared in Example 1 were each transformed into *E. coli* BL21 (DE3), and the enzymes were expressed, purified, and reacted using the same method presented in Example 2-1, and then the reaction products were analyzed using HPLC-UV.

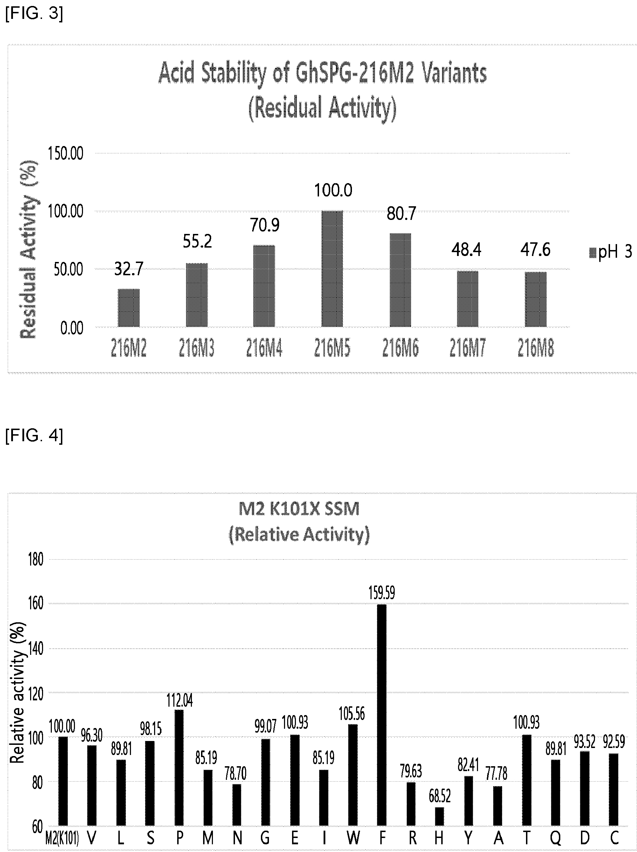

To evaluate acid resistance (residual activity) of GhSPG-216M2 and 6 kinds of variants, 47.5 µL of each purified enzyme was mixed with 2.5 µL of glycine-HCI (1 M, pH 3.0), sodium acetate (1 M, pH 5.0), and Tris-HCl (1 M, pH 7.0), Tris-HCl (1 M, pH 9.0), and incubated at 37°C for 60 minutes. Each of the acid-treated enzyme solutions was used to obtain the residual activity at pH 3 relative to the DON degradation amount at the optimum pH after DON degradation, which is shown in FIG. 3.

As a result, when left at pH 3 and 37°C for 60 minutes, the residual activities of GhSPG-216M2, 216M3, 216M4, 216M5, 216M6, 216M7, and 216M8, relative to those at the optimum pH (highest DON degradation amount), were 32.73%, 55.17%, 70.91%, 100.0%, 80.69%, 48.41%, and 47.58%, respectively. It was confirmed that 216M5, which was a point variant, showed the highest residual activity, and the residual activities of the other five variants were all improved.

### Example 2-4. Comparative evaluation of activity, acid resistance, and thermal tolerance of variants having site-saturated mutagenesis at position 101

To perform comparative evaluation of activity, acid resistance, and thermal tolerance of the variants in which site-saturated mutagenesis was induced in the GhSPG-216M2 sequence, primers were designed and 19 kinds of point mutation and combination variants (hereinafter, referred to as 216M3, 216M4, 216M5, 216M6, 216M7, 216M8, 216M9, 216M10, and 216M11, SEQ ID NOS: 3, 5, 7, 9, 11, 13, 31, 33, and 35) were prepared. The mutation sites based on the amino acid sequence of SEQ ID NO: 1, post-mutation amino acids, and primer sequences for preparing the variants are listed in order in Table 2 below.

**[Table 2]**

| Name of variant | Mutation | | SEQ ID NO. | Primer sequence (5' → 3') |
|---|---|---|---|---|
| 216M12 | K101V | Forward | 37 | GTACAGGAAAGAACTATATTTTTCgtgGATGACGG GAACTACAAGACACGT |
| | | Reverse | 38 | ATCcacGAAAAATATAGTTCTTTCCTGTAC |
| 216M13 | K101L | Forward | 39 | GTACAGGAAAGAACTATATTTTTCctgGATGACGG GAACTACAAGACACGT |
| | | Reverse | 40 | ATCcagGAAAAATATAGTTCTTTCCTGTAC |
| 216M14 | K101S | Forward | 41 | GTACAGGAAAGAACTATATTTTTCagcGATGACG GGAACTACAAGACACGT |
| | | Reverse | 42 | ATCgctGAAAAATATAGTTCTTTCCTGTAC |
| 216M15 | K101P | Forward | 43 | GTACAGGAAAGAACTATATTTTTCccgGATGACGG GAACTACAAGACACGT |
| | | Reverse | 44 | ATCcggGAAAAATATAGTTCTTTCCTGTAC |
| 216M16 | K101M | Forward | 45 | GTACAGGAAAGAACTATATTTTTCatgGATGACGG GAACTACAAGACACGT |
| | | Reverse | 46 | ATCcatGAAAAATATAGTTCTTTCCTGTAC |
| 216M17 | K101N | Forward | 47 | GTACAGGAAAGAACTATATTTTTCaacGATGACG GGAACTACAAGACACGT |
| | | Reverse | 48 | ATCgttGAAAAATATAGTTCTTTCCTGTAC |
| 216M18 | K101G | Forward | 49 | GTACAGGAAAGAACTATATTTTTCggcGATGACG GGAACTACAAGACACGT |
| | | Reverse | 50 | ATCgccGAAAAATATAGTTCTTTCCTGTAC |
| 216M19 | K101E | Forward | 51 | GTACAGGAAAGAACTATATTTTTCgaaGATGACG GGAACTACAAGACACGT |
| | | Reverse | 52 | ATCttcGAAAAATATAGTTCTTTCCTGTAC |
| 216M20 | K101I | Forward | 53 | GTACAGGAAAGAACTATATTTTTCattGATGACGG GAACTACAAGACACGT |
| | | Reverse | 54 | ATCaatGAAAAATATAGTTCTTTCCTGTAC |
| 216M21 | K101W | Forward | 55 | GTACAGGAAAGAACTATATTTTTCtggGATGACGG GAACTACAAGACACGT |
| | | Reverse | 56 | ATCccaGAAAAATATAGTTCTTTCCTGTAC |
| 216M22 | K101F | Forward | 57 | GTACAGGAAAGAACTATATTTTTCtttGATGACGG GAACTACAAGACACGT |
| | | Reverse | 58 | ATCaaaGAAAAATATAGTTCTTTCCTGTAC |
| 216M23 | K101R | Forward | 59 | GTACAGGAAAGAACTATATTTTTCcgtGATGACGG GAACTACAAGACACGT |
| | | Reverse | 60 | ATCacgGAAAAATATAGTTCTTTCCTGTAC |
| 216M24 | K101H | Forward | 61 | GTACAGGAAAGAACTATATTTTTCcatGATGACGG GAACTACAAGACACGT |
| | | Reverse | 62 | ATCatgGAAAAATATAGTTCTTTCCTGTAC |
| 216M25 | K101Y | Forward | 63 | GTACAGGAAAGAACTATATTTTTCtatGATGACGG GAACTACAAGACACGT |
| | | Reverse | 64 | ATCataGAAAAATATAGTTCTTTCCTGTAC |
| 216M26 | K101A | Forward | 65 | GTACAGGAAAGAACTATATTTTTCgcgGATGACG GGAACTACAAGACACGT |
| | | Reverse | 66 | ATCcgcGAAAAATATAGTTCTTTCCTGTAC |
| 216M27 | K101T | Forward | 67 | GTACAGGAAAGAACTATATTTTTCaccGATGACGG GAACTACAAGACACGT |
| | | Reverse | 68 | ATCggtGAAAAATATAGTTCTTTCCTGTAC |
| 216M28 | K101Q | Forward | 69 | GTACAGGAAAGAACTATATTTTTCcagGATGACG GGAACTACAAGACACGT |
| | | Reverse | 70 | ATCctgGAAAAATATAGTTCTTTCCTGTAC |
| 216M29 | K101D | Forward | 71 | GTACAGGAAAGAACTATATTTTTCgatGATGACGG GAACTACAAGACACGT |
| | | Reverse | 72 | ATCatcGAAAAATATAGTTCTTTCCTGTAC |
| 216M30 | K101C | Forward | 73 | GTACAGGAAAGAACTATATTTTTCtgcGATGACGG GAACTACAAGACACGT |
| | | Reverse | 74 | ATCgcaGAAAAATATAGTTCTTTCCTGTAC |

In detail, 19 kinds of the point mutations at position 101 were prepared by PCR using the polynucleotide encoding GhSPG-216M2 (SEQ ID NO: 1) prepared in Example 1-1, which was cloned into the pET vector, as a template, primers (SEQ ID NOS: 37 to 74 of Table 2), and a PCR premix (iNtRON, cat no. 25185). PCR was performed using an Eppendorf Mastercycler Nexus GX2, and the reaction conditions are as follows:
Initial denaturation - 94°C, 2 min
Denaturation - 94°C, 20 sec
Annealing - 50°C, 10 sec
Extension - 72°C, 8 min (15 cycles from denaturation to extension)
Final Extension - 72°C, 5 min

The prepared truncated variants were ligated using a QuickChange Site-Directed Mutagenesis kit (Agilent, Cat# 200518), and then transformed into the E. *coli* Dh5α strain to identify the presence of sequence mutations through sequencing.

The activity, thermal tolerance, and acid resistance of 19 kinds of variants were evaluated using the same methods as in Examples 2-1, 2-2, and 2-3, respectively, and the results are shown in FIGS. 4 to 6.

In detail, in FIG. 4, it was confirmed that when the amino acid (K; Lys) at position 101 of GhSPG-216 M2 was substituted with another amino acid, the activity of the variant was improved by about 0.9% to 12% when the enzyme activity was expressed as a relative ratio.

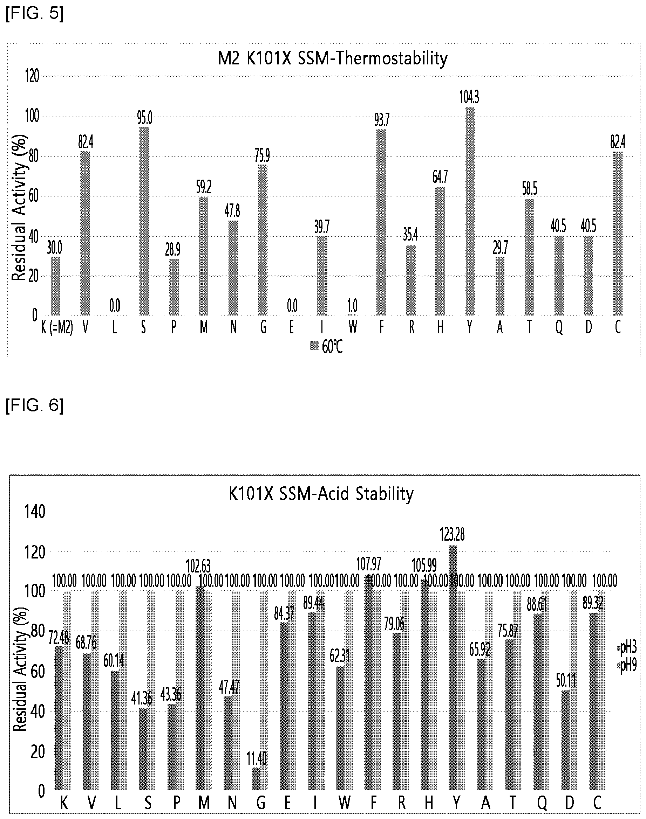

Further, in FIG. 5, it was confirmed that when the amino acid at position 101 of GhSPG-216 M2 was substituted with another amino acid, the thermal stability was improved by about 4% to 22% at 50°C and by about 10% to 75% at 60°C.

In FIG. 6, it was confirmed that when the amino acid at position 101 of GhSPG-216 M2 was substituted with another amino acid, the acid resistance was improved by 3% to 51%.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within the metes and bounds of the claims, or equivalents of such metes and bounds are therefore intended to be embraced by the claims.

### [Sequence Listing]

| SEQ ID NO. | Sequence Name | SEQUENCES |
|---|---|---|
| 1 | GhSPG-216M2 AA | MASSGPKESAANNPGLQTPPDEATKGYFMQQTMFRIKDPK VSLDFYSRVLGMSLLKRIDVPDMKMTLYFMGYEDVSSAPSD PIERTAWTFGRPATIELTHKWGTESDPEFKGYHNGNSEPIGF GHIGITVDDTYKACERFERLGVEFVKKPDDGYAFIKDPDGYW IEIFDLKNIRNVVNGVA |
| 2 | GhSPG-216M2 NT | ATGGCATCGTCCGGGCCGAAAGAGTCGGCTGCCAATAATC CTGGCTTGCAAACTCCACCTGACGAGGCCACTAAGGGTTA TTTTATGCAACAGACAATGTTTCGCATTAAAGACCCTAAAG TGTCTTTGGACTTCTATTCGCGCGTGCTTGGTATGAGCCTT TTGAAAAGAATTGACGTCCCGGATATGAAGatgACGTTATAT TTTATGGGATATGAAGATGTAAGTAGTGCGCCAAGTGATCC AATAGAGCGGACCGCCTGGACATTTGGCAGACCAGCGAC AATCGAATTGACCCATAAGTGGGGTACCGAGAGCGACCCG GAGTTTAAAGGTTACCATAATGGCAATTCTGAACCAattGGT |
| | | TTCGGGCACATAGGCATCACGGTCGATGACACTTACAAGG CGTGTGAGAGATTCGAACGTCTTGGGGTAGAATTTGTGAA AAAGCCGGATGACGGCTATGCGTTTATTAAAGACCCCGAT GGCTACTGGATAGAGATATTTGACTTGAAGAATATACGCAA TGTGGTAAATGGAGTCGCT |
| 3 | 216M3 AA | MASSGPKESAANNPGLQTPPDEATKGYIMQQTMFRIKDPKV SLDFYSRVLGMSLLKRIDVPDMKMTLYFMGYEDVSSAPSDPI ERTAWTFGRPATIELTHKWGTESDPEFKGYHNGNSEPIGFG HIGITVDDTYKACERFERLGVEFVKKPDDGYAFIKDPDGYWI EIFDLKNIRNVVNGVA |
| 4 | 216M3 NT | ATGGCATCGTCCGGGCCGAAAGAGTCGGCTGCCAATAATC CTGGCTTGCAAACTCCACCTGACGAGGCCACTAAGGGTTA TattATGCAACAGACAATGTTTCGCATTAAAGACCCTAAAGT GTCTTTGGACTTCTATTCGCGCGTGCTTGGTATGAGCCTTT TGAAAAGAATTGACGTCCCGGATATGAAGatgACGTTATATT TTATGGGATATGAAGATGTAAGTAGTGCGCCAAGTGATCCA ATAGAGCGGACCGCCTGGACATTTGGCAGACCAGCGACA ATCGAATTGACCCATaaaTGGGGTACCGAGAGCGACCCGG AGTTTAAAGGTTACCATAATGGCAATTCTGAACCAattGGTTT CGGGCACATAGGCATCACGGTCGATGACACTTACAAGGC GTGTGAGAGATTCGAACGTCTTGGGGTAGAATTTGTGAAA AAGCCGgatGACGGCTATGCGTTTATTAAAGACCCCGATGG CTACTGGATAGAGATATTTGACTTGAAGAATATACGCAATGT GGTAAATGGAGTCGCT |
| 5 | 216M4 AA | MASSGPKESAANNPGLQTPPDEATKGYFMQQTMFRIKDPK VSLDFYSRVLGMSLLKRIDVPDMKMTLYFMGYEDVSSAPSD |
| | | PIERTAWTFGRPATIELTHFWGTESDPEFKGYHNGNSEPIGF GHIGITVDDTYKACERFERLGVEFVKKPDDGYAFIKDPDGYW IEIFDLKNIRNVVNGVA |
| 6 | 216M4 NT | ATGGCATCGTCCGGGCCGAAAGAGTCGGCTGCCAATAATC CTGGCTTGCAAACTCCACCTGACGAGGCCACTAAGGGTTA TtttATGCAACAGACAATGTTTCGCATTAAAGACCCTAAAGTG TCTTTGGACTTCTATTCGCGCGTGCTTGGTATGAGCCTTTT GAAAAGAATTGACGTCCCGGATATGAAGatgACGTTATATTT TATGGGATATGAAGATGTAAGTAGTGCGCCAAGTGATCCAA TAGAGCGGACCGCCTGGACATTTGGCAGACCAGCGACAA TCGAATTGACCCATtttTGGGGTACCGAGAGCGACCCGGAG TTTAAAGGTTACCATAATGGCAATTCTGAACCAattGGTTTCG GGCACATAGGCATCACGGTCGATGACACTTACAAGGCGTG TGAGAGATTCGAACGTCTTGGGGTAGAATTTGTGAAAAAG CCGgatGACGGCTATGCGTTTATTAAAGACCCCGATGGCTA CTGGATAGAGATATTTGACTTGAAGAATATACGCAATGTGG TAAATGGAGTCGCT |
| 7 | 216M5 AA | MASSGPKESAANNPGLQTPPDEATKGYFMQQTMFRIKDPK VSLDFYSRVLGMSLLKRIDVPDMKMTLYFMGYEDVSSAPSD PIERTAWTFGRPATIELTHKWGTESDPEFKGYHNGNSEPIGF GHIGITVDDTYKACERFERLGVEFVKKPGDGYAFIKDPDGYW IEIFDLKNIRNVVNGVA |
| 8 | 216M5 NT | ATGGCATCGTCCGGGCCGAAAGAGTCGGCTGCCAATAATC CTGGCTTGCAAACTCCACCTGACGAGGCCACTAAGGGTTA TTTTATGCAACAGACAATGTTTCGCATTAAAGACCCTAAAG TGTCTTTGGACTTCTATTCGCGCGTGCTTGGTATGAGCCTT |
| | | TTGAAAAGAATTGACGTCCCGGATATGAAGatgACGTTATAT TTTATGGGATATGAAGATGTAAGTAGTGCGCCAAGTGATCC AATAGAGCGGACCGCCTGGACATTTGGCAGACCAGCGAC AATCGAATTGACCCATAAGTGGGGTACCGAGAGCGACCCG GAGTTTAAAGGTTACCATAATGGCAATTCTGAACCAattGGT TTCGGGCACATAGGCATCACGGTCGATGACACTTACAAGG CGTGTGAGAGATTCGAACGTCTTGGGGTAGAATTTGTGAA AAAGCCGggcGACGGCTATGCGTTTATTAAAGACCCCGATG GCTACTGGATAGAGATATTTGACTTGAAGAATATACGCAAT GTGGTAAATGGAGTCGCT |
| 9 | 216M6 AA | MASSGPKESAANNPGLQTPPDEATKGYIMQQTMFRIKDPKV SLDFYSRVLGMSLLKRIDVPDMKMTLYFMGYEDVSSAPSDPI ERTAWTFGRPATIELTHFWGTESDPEFKGYHNGNSEPIGFG HIGITVDDTYKACERFERLGVEFVKKPDDGYAFIKDPDGYWI EIFDLKNIRNVVNGVA |
| 10 | 216M6 NT | ATGGCATCGTCCGGGCCGAAAGAGTCGGCTGCCAATAATC CTGGCTTGCAAACTCCACCTGACGAGGCCACTAAGGGTTA TattATGCAACAGACAATGTTTCGCATTAAAGACCCTAAAGT GTCTTTGGACTTCTATTCGCGCGTGCTTGGTATGAGCCTTT TGAAAAGAATTGACGTCCCGGATATGAAGatgACGTTATATT TTATGGGATATGAAGATGTAAGTAGTGCGCCAAGTGATCCA ATAGAGCGGACCGCCTGGACATTTGGCAGACCAGCGACA ATCGAATTGACCCATtttTGGGGTACCGAGAGCGACCCGGA GTTTAAAGGTTACCATAATGGCAATTCTGAACCAattGGTTTC GGGCACATAGGCATCACGGTCGATGACACTTACAAGGCGT GTGAGAGATTCGAACGTCTTGGGGTAGAATTTGTGAAAAA |
| | | GCCGgatGACGGCTATGCGTTTATTAAAGACCCCGATGGCT ACTGGATAGAGATATTTGACTTGAAGAATATACGCAATGTG GTAAATGGAGTCGCT |
| 11 | 216M7 AA | MASSGPKESAANNPGLQTPPDEATKGYIMQQTMFRIKDPKV SLDFYSRVLGMSLLKRIDVPDMKMTLYFMGYEDVSSAPSDPI ERTAWTFGRPATIELTHKWGTESDPEFKGYHNGNSEPIGFG HIGITVDDTYKACERFERLGVEFVKKPGDGYAFIKDPDGYWI EIFDLKNIRNVVNGVA |
| 12 | 216M7 NT | ATGGCATCGTCCGGGCCGAAAGAGTCGGCTGCCAATAATC CTGGCTTGCAAACTCCACCTGACGAGGCCACTAAGGGTTA TattATGCAACAGACAATGTTTCGCATTAAAGACCCTAAAGT GTCTTTGGACTTCTATTCGCGCGTGCTTGGTATGAGCCTTT TGAAAAGAATTGACGTCCCGGATATGAAGatgACGTTATATT TTATGGGATATGAAGATGTAAGTAGTGCGCCAAGTGATCCA ATAGAGCGGACCGCCTGGACATTTGGCAGACCAGCGACA ATCGAATTGACCCATaaaTGGGGTACCGAGAGCGACCCGG AGTTTAAAGGTTACCATAATGGCAATTCTGAACCAattGGTTT CGGGCACATAGGCATCACGGTCGATGACACTTACAAGGC GTGTGAGAGATTCGAACGTCTTGGGGTAGAATTTGTGAAA AAGCCGggcGACGGCTATGCGTTTATTAAAGACCCCGATGG CTACTGGATAGAGATATTTGACTTGAAGAATATACGCAATGT GGTAAATGGAGTCGCT |
| 13 | 216M8 AA | MASSGPKESAANNPGLQTPPDEATKGYIMQQTMFRIKDPKV SLDFYSRVLGMSLLKRIDVPDMKMTLYFMGYEDVSSAPSDPI ERTAWTFGRPATIELTHFWGTESDPEFKGYHNGNSEPIGFG HIGITVDDTYKACERFERLGVEFVKKPGDGYAFIKDPDGYWI |
| | | EIFDLKNIRNVVNGVA |
| 14 | 216M8 NT | ATGGCATCGTCCGGGCCGAAAGAGTCGGCTGCCAATAATC CTGGCTTGCAAACTCCACCTGACGAGGCCACTAAGGGTTA TattATGCAACAGACAATGTTTCGCATTAAAGACCCTAAAGT GTCTTTGGACTTCTATTCGCGCGTGCTTGGTATGAGCCTTT TGAAAAGAATTGACGTCCCGGATATGAAGatgACGTTATATT TTATGGGATATGAAGATGTAAGTAGTGCGCCAAGTGATCCA ATAGAGCGGACCGCCTGGACATTTGGCAGACCAGCGACA ATCGAATTGACCCATtttTGGGGTACCGAGAGCGACCCGGA GTTTAAAGGTTACCATAATGGCAATTCTGAACCAattGGTTTC GGGCACATAGGCATCACGGTCGATGACACTTACAAGGCGT GTGAGAGATTCGAACGTCTTGGGGTAGAATTTGTGAAAAA GCCGgggGACGGCTATGCGTTTATTAAAGACCCCGATGGCT ACTGGATAGAGATATTTGACTTGAAGAATATACGCAATGTG GTAAATGGAGTCGCT |
| 25 | 216M9-F | GGCCACTAAGGGTTATTTTctgCAACAGACAATGTTTCGCAT TAAAGAC |
| 26 | 216M9-R | cagAAAATAACCCTTAGTGGCCTCGTCAGGTGGAGTTTGCA AGCC |
| 27 | 216M10-F | AGCGGACCattTGGACATTTGGCAGACCAGCGACAATC |
| 28 | 216M10-R | AAATGTCCAaatGGTCCGCTCTATTGGATCACTTGGCGC |
| 29 | 216M11-F | CCTGGACATTTagcAGACCAGCGACAATCGAATTGACCC |
| 30 | 216M11-R | TGGTCTgctAAATGTCCAGGCGGTCCGCTCTATTGGATC |
| 15 | 216M3-F | GGCCACTAAGGGTTATattATGCAACAGACAATGTTTCGCAT |
| 16 | 216M3-R | CATaatATAACCCTTAGTGGCCTCGTCAGGTGGAGTTTGCA AG |
| 17 | 216M4-F | ATtttTGGGGTACCGAGAGCGACCCGGAGTTTAAAGGTTAC |
| 18 | 216M4-R | GCTCTCGGTACCCCAaaaATGGGTCAATTCGATTGTCGC |
| 19 | 216M5-F | AGAATTTGTGAAAAAGCCGggcGACGGCTATGCGTTTATTA AAGACCC |
| 20 | 216M5-R | gccCGGCTTTTTCACAAATTCTACCCCAAGACGTTCGAATCT CTCACAC |
| 21 | GhSPG-216 AA | MASSGPKESAANNPGLQTPPDEATKGYFMQQTMFRIKDPK VSLDFYSRVLGMSLLKRIDVPDMKFTLYFMGYEDVSSAPSDP IERTAWTFGRPATIELTHKWGTESDPEFKGYHNGNSEPRGF GHIGITVDDTYKACERFERLGVEFVKKPDDGYAFIKDPDGYW IEIFDLKNIRNVVNGVA |
| 22 | GhSPG-216 NT | ATGGCATCGTCCGGGCCGAAAGAGTCGGCTGCCAATAATC CTGGCTTGCAAACTCCACCTGACGAGGCCACTAAGGGTTA TTTTATGCAACAGACAATGTTTCGCATTAAAGACCCTAAAG TGTCTTTGGACTTCTATTCGCGCGTGCTTGGTATGAGCCTT TTGAAAAGAATTGACGTCCCGGATATGAAGTTCACGTTATA TTTTATGGGATATGAAGATGTAAGTAGTGCGCCAAGTGATC CAATAGAGCGGACCGCCTGGACATTTGGCAGACCAGCGA CAATCGAATTGACCCATAAGTGGGGTACCGAGAGCGACCC GGAGTTTAAAGGTTACCATAATGGCAATTCTGAACCACGG GGTTTCGGGCACATAGGCATCACGGTCGATGACACTTACA AGGCGTGTGAGAGATTCGAACGTCTTGGGGTAGAATTTGT GAAAAAGCCGGATGACGGCTATGCGTTTATTAAAGACCCC GATGGCTACTGGATAGAGATATTTGACTTGAAGAATATACG CAATGTGGTAAATGGAGTCGCT |
| 23 | GhSPG-9 | MGSLDSKESPANNPGLHSPPDEATKGYILQQTMFRIKDPKPA |
| | AA | LEFYSRVLGMSLLNKVDVPYMKMTLYMMGYEDVSSAPSDP VEKTIWTFGRPATMELTHFWGTENDPEFKGYHDGNSEPTGF GHIGITVDDMYKACERFESLGVEFVKKPGDGYAFIKDPDGY WIEIFDLNGIRAIVNNLA |
| 24 | GhSPG-9 NT | ATGGGGTCCCTTGATAGCAAAGAATCGCCCGCTAATAACC CTGGACTTCATAGTCCCCCCGACGAGGCCACAAAAGGCTA CATCCTTCAACAAACTATGTTCCGCATAAAAGACCCGAAGC CAGCACTGGAGTTCTATTCTAGAGTATTGGGGATGTCATTA CTGAACAAAGTGGATGTACCATACATGAAAATGACCCTGTA CATGATGGGTTACGAAGATGTTTCATCTGCCCCAAGCGAT CCCGTCGAGAAGACTATTTGGACGTTCGGCCGTCCTGCA ACGATGGAACTGACCCATTTTTGGGGGACCGAGAATGACC CCGAATTTAAGGGTTACCATGACGGGAACTCAGAACCTAC CGGTTTTGGCCATATTGGTATTACTGTGGATGATATGTATAA AGCGTGCGAGCGGTTTGAGAGCCTTGGTGTTGAATTTGTT AAAAAACCAGGAGACGGATATGCTTTTATCAAGGACCCGG ACGGCTACTGGATTGAGATTTTTGATTTGAACGGAATACGG GCTATCGTAAATAACTTAGCA |
| 31 | 216M9 AA | MASSGPKESAANNPGLQTPPDEATKGYFLQQTMFRIKDPKV SLDFYSRVLGMSLLKRIDVPDMKMTLYFMGYEDVSSAPSDPI ERTAWTFGRPATIELTHKWGTESDPEFKGYHNGNSEPIGFG HIGITVDDTYKACERFERLGVEFVKKPDDGYAFIKDPDGYWI EIFDLKNIRNVVNGVA |
| 32 | 216M9 NT | ATGGCATCGTCCGGGCCGAAAGAGTCGGCTGCCAATAATC CTGGCTTGCAAACTCCACCTGACGAGGCCACTAAGGGTTA TTTTctgCAACAGACAATGTTTCGCATTAAAGACCCTAAAGT |
| | | GTCTTTGGACTTCTATTCGCGCGTGCTTGGTATGAGCCTTT TGAAAAGAATTGACGTCCCGGATATGAAGatgACGTTATATT TTATGGGATATGAAGATGTAAGTAGTGCGCCAAGTGATCCA ATAGAGCGGACCGCCTGGACATTTGGCAGACCAGCGACA ATCGAATTGACCCATAAGTGGGGTACCGAGAGCGACCCG GAGTTTAAAGGTTACCATAATGGCAATTCTGAACCAattGGT TTCGGGCACATAGGCATCACGGTCGATGACACTTACAAGG CGTGTGAGAGATTCGAACGTCTTGGGGTAGAATTTGTGAA AAAGCCGGATGACGGCTATGCGTTTATTAAAGACCCCGAT GGCTACTGGATAGAGATATTTGACTTGAAGAATATACGCAA TGTGGTAAATGGAGTCGCT |
| 33 | 216M10 AA | MASSGPKESAANNPGLQTPPDEATKGYFMQQTMFRIKDPK VSLDFYSRVLGMSLLKRIDVPDMKMTLYFMGYEDVSSAPSD PIERTIWTFGRPATIELTHKWGTESDPEFKGYHNGNSEPIGF GHIGITVDDTYKACERFERLGVEFVKKPDDGYAFIKDPDGYW IEIFDLKNIRNVVNGVA |
| 34 | 216M10 NT | ATGGCATCGTCCGGGCCGAAAGAGTCGGCTGCCAATAATC CTGGCTTGCAAACTCCACCTGACGAGGCCACTAAGGGTTA TTTTATGCAACAGACAATGTTTCGCATTAAAGACCCTAAAG TGTCTTTGGACTTCTATTCGCGCGTGCTTGGTATGAGCCTT TTGAAAAGAATTGACGTCCCGGATATGAAGatgACGTTATAT TTTATGGGATATGAAGATGTAAGTAGTGCGCCAAGTGATCC AATAGAGCGGACCattTGGACATTTGGCAGACCAGCGACAA TCGAATTGACCCATAAGTGGGGTACCGAGAGCGACCCGG AGTTTAAAGGTTACCATAATGGCAATTCTGAACCAattGGTTT CGGGCACATAGGCATCACGGTCGATGACACTTACAAGGC |
| | | GTGTGAGAGATTCGAACGTCTTGGGGTAGAATTTGTGAAA AAGCCGGATGACGGCTATGCGTTTATTAAAGACCCCGATG GCTACTGGATAGAGATATTTGACTTGAAGAATATACGCAAT GTGGTAAATGGAGTCGCT |
| 35 | 216M11 AA | MASSGPKESAANNPGLQTPPDEATKGYFMQQTMFRIKDPK VSLDFYSRVLGMSLLKRIDVPDMKMTLYFMGYEDVSSAPSD PIERTAWTFSRPATIELTHKWGTESDPEFKGYHNGNSEPIGF GHIGITVDDTYKACERFERLGVEFVKKPDDGYAFIKDPDGYW IEIFDLKNIRNVVNGVA |
| 36 | 216M11 NT | ATGGCATCGTCCGGGCCGAAAGAGTCGGCTGCCAATAATC CTGGCTTGCAAACTCCACCTGACGAGGCCACTAAGGGTTA TTTTATGCAACAGACAATGTTTCGCATTAAAGACCCTAAAG TGTCTTTGGACTTCTATTCGCGCGTGCTTGGTATGAGCCTT TTGAAAAGAATTGACGTCCCGGATATGAAGatgACGTTATAT TTTATGGGATATGAAGATGTAAGTAGTGCGCCAAGTGATCC AATAGAGCGGACCGCCTGGACATTTagcAGACCAGCGACA ATCGAATTGACCCATAAGTGGGGTACCGAGAGCGACCCG GAGTTTAAAGGTTACCATAATGGCAATTCTGAACCAattGGT TTCGGGCACATAGGCATCACGGTCGATGACACTTACAAGG CGTGTGAGAGATTCGAACGTCTTGGGGTAGAATTTGTGAA AAAGCCGGATGACGGCTATGCGTTTATTAAAGACCCCGAT GGCTACTGGATAGAGATATTTGACTTGAAGAATATACGCAA TGTGGTAAATGGAGTCGCT |
| 37 | K101V-F | GTACAGGAAAGAACTATATTTTTCgtgGATGACGGGAACTAC AAGACACGT |
| 38 | K101V-R | ATCcacGAAAAATATAGTTCTTTCCTGTAC |
| 39 | K101L-F | GTACAGGAAAGAACTATATTTTTCctgGATGACGGGAACTAC AAGACACGT |
| 40 | K101L-R | ATCcagGAAAAATATAGTTCTTTCCTGTAC |
| 41 | K101S-F | GTACAGGAAAGAACTATATTTTTCagcGATGACGGGAACTAC AAGACACGT |
| 42 | K101S-R | ATCgctGAAAAATATAGTTCTTTCCTGTAC |
| 43 | K101P-F | GTACAGGAAAGAACTATATTTTTCccgGATGACGGGAACTAC AAGACACGT |
| 44 | K101P-R | ATCcggGAAAAATATAGTTCTTTCCTGTAC |
| 45 | K101M-F | GTACAGGAAAGAACTATATTTTTCatgGATGACGGGAACTAC AAGACACGT |
| 46 | K101M-R | ATCcatGAAAAATATAGTTCTTTCCTGTAC |
| 47 | K101N-F | GTACAGGAAAGAACTATATTTTTCaacGATGACGGGAACTAC AAGACACGT |
| 48 | K101N-R | ATCgttGAAAAATATAGTTCTTTCCTGTAC |
| 49 | K101G-F | GTACAGGAAAGAACTATATTTTTCggcGATGACGGGAACTAC AAGACACGT |
| 50 | K101G-R | ATCgccGAAAAATATAGTTCTTTCCTGTAC |
| 51 | K101E-F | GTACAGGAAAGAACTATATTTTTCgaaGATGACGGGAACTA CAAGACACGT |
| 52 | K101E-R | ATCttcGAAAAATATAGTTCTTTCCTGTAC |
| 53 | K101I-F | GTACAGGAAAGAACTATATTTTTCattGATGACGGGAACTAC AAGACACGT |
| 54 | K101I-R | ATCaatGAAAAATATAGTTCTTTCCTGTAC |
| 55 | K101W-F | GTACAGGAAAGAACTATATTTTTCtggGATGACGGGAACTAC AAGACACGT |
| 56 | K101W-R | ATCccaGAAAAATATAGTTCTTTCCTGTAC |
| 57 | K101F-F | GTACAGGAAAGAACTATATTTTTCtttGATGACGGGAACTACA AGACACGT |
| 58 | K101F-R | ATCaaaGAAAAATATAGTTCTTTCCTGTAC |
| 59 | K101R-F | GTACAGGAAAGAACTATATTTTTCcgtGATGACGGGAACTAC AAGACACGT |
| 60 | K101R-R | ATCacgGAAAAATATAGTTCTTTCCTGTAC |
| 61 | K101H-F | GTACAGGAAAGAACTATATTTTTCcatGATGACGGGAACTAC AAGACACGT |
| 62 | K101H-R | ATCatgGAAAAATATAGTTCTTTCCTGTAC |
| 63 | K101Y-F | GTACAGGAAAGAACTATATTTTTCtatGATGACGGGAACTAC AAGACACGT |
| 64 | K101Y-R | ATCataGAAAAATATAGTTCTTTCCTGTAC |
| 65 | K101A-F | GTACAGGAAAGAACTATATTTTTCgcgGATGACGGGAACTAC AAGACACGT |
| 66 | K101A-R | ATCcgcGAAAAATATAGTTCTTTCCTGTAC |
| 67 | K101T-F | GTACAGGAAAGAACTATATTTTTCaccGATGACGGGAACTAC AAGACACGT |
| 68 | K101T-R | ATCggtGAAAAATATAGTTCTTTCCTGTAC |
| 69 | K101Q-F | GTACAGGAAAGAACTATATTTTTCcagGATGACGGGAACTAC AAGACACGT |
| 70 | K101Q-R | ATCctgGAAAAATATAGTTCTTTCCTGTAC |
| 71 | K101D-F | GTACAGGAAAGAACTATATTTTTCgatGATGACGGGAACTAC AAGACACGT |
| 72 | K101D-R | ATCatcGAAAAATATAGTTCTTTCCTGTAC |
| 73 | K101C-F | GTACAGGAAAGAACTATATTTTTCtgcGATGACGGGAACTAC AAGACACGT |
| 74 | K101C-R | ATCgcaGAAAAATATAGTTCTTTCCTGTAC |

## Claims

1. A modified polypeptide having deoxynivalenol (DON) degradation activity, wherein amino acids at positions corresponding to any one or more positions selected from positions 28, 29, 87, 91, 101, and 152 of SEQ **ID** NO: 1 are substituted with different amino acids.

2. The modified polypeptide of claim 1, wherein an amino acid corresponding to position 28 of SEQ **ID** NO: 1 is substituted with isoleucine; an amino acid corresponding to position 29 of SEQ **ID** NO: 1 is substituted with leucine; an amino acid corresponding to position 87 of SEQ **ID** NO: 1 is substituted with isoleucine; an amino acid corresponding to position 91 of SEQ ID NO: 1 is substituted with serine; an amino acid corresponding to position 101 of SEQ ID NO: 1 is substituted with phenylalanine; or an amino acid corresponding to position 152 of SEQ ID NO: 1 is substituted with glycine.

3. The modified polypeptide of claim 1, wherein an amino acid corresponding to position 28 of SEQ ID NO: 1 is substituted with isoleucine; and an amino acid corresponding to position 101 of SEQ ID NO: 1 is substituted with phenylalanine.

4. The modified polypeptide of claim 1, wherein an amino acid corresponding to position 28 of SEQ ID NO: 1 is substituted with isoleucine; and an amino acid corresponding to position 152 of SEQ ID NO: 1 is substituted with glycine.

5. The modified polypeptide of claim 1, wherein an amino acid corresponding to position 28 of SEQ ID NO: 1 is substituted with isoleucine; an amino acid corresponding to position 101 of SEQ ID NO: 1 is substituted with phenylalanine; and an amino acid corresponding to position 152 of SEQ ID NO: 1 is substituted with glycine.

6. The modified polypeptide of claim 1, comprising any one selected from SEQ ID NO: 3, SEQ ID NO: 5, SEQ ID NO: 7, SEQ ID NO: 9, SEQ ID NO: 11, SEQ ID NO: 13, SEQ ID NO: 31, SEQ ID NO: 33, and SEQ ID NO: 35.

7. The modified polypeptide of claim 1, wherein one or more properties selected from DON degradation activity, thermal tolerance, thermal stability, and acid resistance are improved, as compared to the polypeptide consisting of the amino acid sequence of SEQ ID NO: 1.

8. A polynucleotide encoding the modified polypeptide of any one of claims 1 to 7.

9. A microorganism comprising any one or more of the modified polypeptide of any one of claims 1 to 7; and a polynucleotide encoding the modified polypeptide.

10. A composition for degrading deoxynivalenol (DON), the composition comprising any one or more of the modified polypeptide of any one of claims 1 to 7; and a microorganism expressing the modified polypeptide.

11. A feed additive composition comprising any one or more of the modified polypeptide of any one of claims 1 to 7; and a microorganism expressing the modified polypeptide.

12. A method of degrading deoxynivalenol (DON), the method comprising the step of reacting DON with any one or more of the modified polypeptide of any one of claims 1 to 7; and a microorganism expressing the modified polypeptide.

13. A method of preparing a feed product, the method comprising the step of mixing a feed component with the feed additive composition of claim 11.

14. A method of preparing a modified polypeptide having DON degradation activity, the method comprising the steps of:
culturing the microorganism of claim 9; and
recovering the modified polypeptide having DON degradation activity of any one of claims 1 to 7, which is expressed in the culturing step.

15. A use of the modified polypeptide of any one of claims 1 to 7 for the degradation of DON; and a use for the degradation of DON by one or more microorganisms expressing the modified polypeptide.
